# EUROPÄISCHE PATENTANMELDUNG

(11) **EP 0 524 146 A1**
(43) Veröffentlichungstag der Anmeldung: **20.01.1993**
(21) Anmeldenummer: 92810525.3
(22) Anmeldetag: 10.07.1992
(51) Int. Cl.: C07D 295/18, A61K 31/495

(54) **Aminosubstituierte Piperazinderivate**

(30) Priorität: 19.07.1991 CH 2160/91; 27.04.1992 CH 1340/92
(71) Anmelder: CIBA-GEIGY AG, CH-4002 Basel (CH)
(72) Erfinder: Ferrini, Pier Giorgio, Dr., CH-4102 Binningen (CH)

(57) **Zusammenfassung**

Aminosubstituierte Piperazinderivate der Formel I, 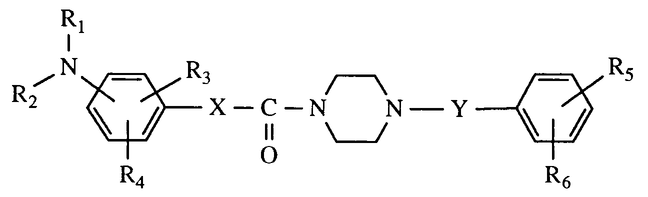
worin R₁ - R₆, X und Y wie in der Beschreibung definiert sind, sowie Salze davon, weisen die Biosynthese von Interleukin-1 (IL-1) hemmende sowie analgetische Eigenschaften auf und können daher als Arzneimittelwirkstoffe verwendet werden. Sie werden in an sich bekannter Weise hergestellt.

## Beschreibung

Die Erfindung betrifft neue aminosubstituierte Piperazinderivate der Formel I, 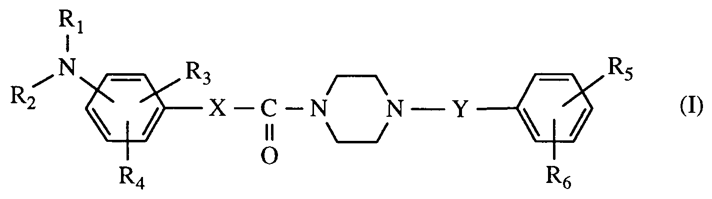
worin R₁ für Wasserstoff, Niederalkyl, Niederalkoxy-niederalkyl, Niederalkoxy-niederalkoxy-niederalkyl, Aryloxy-niederalkyl, Arylniederalkoxy-niederalkyl, N-Niederalkyl-amino-niederalkyl oder N,N-Diniederalkylamino-niederalkyl steht, R₂ Niederalkenoyl, (Carboxy oder Niederalkoxycarbonyl)-niederalkenoyl, elektronegativ substituiertes Niederalkanoyl, Carbamoyl, N-Niederalkylcarbamoyl, N,N-Diniederalkylcarbamoyl, Carboxy-carbonyl, Niederalkoxycarbonyl-carbonyl, (Carbamoyl, N-Niederalkylcarbamoyl oder N,N-Diniederalkylcarbamoyl)-carbonyl bedeutet oder R₂, sofern R₁ für Niederalkoxy-niederalkyl, Niederalkoxy-niederalkoxy-niederalkyl, Aryloxy-niederalkyl, Arylniederalkoxy-niederalkyl, N-Niederalkylamino-niederalkyl oder N,N-Diniederalkylamino-niederalkyl steht, auch Wasserstoff, Niederalkanoyl, Carboxy, Niederalkoxycarbonyl oder Arylniederalkoxycarbonyl bedeuten kann, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, und X und Y unabhängig voneinander eine direkte Bindung, Niederalkylen oder Niederalkenylen bedeuten, und Salze davon, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Elektronegativ substituiertes Niederalkanoyl bedeutet z.B. Niederalkanoyl, das durch Halogen, Amino, Niederalkylamino, (Carboxy oder Niederalkoxycarbonyl)-niederalkylamino, Diniederalkylamino; 4- bis 6-gliedriges Niederalkylenamino, z.B. Piperidino; Morpholino, Thiomorpholino, Piperazino - gegebenenfalls in 4-Stellung Niederalkyl- oder Niederalkanoyl-substituiert -, Hydroxy, Niederalkoxy, Acyloxy, Niederalkylthio, Niederalkylsulfinyl, Niederalkylsulfonyl, Arylthio, Arylsulfinyl, Arylsulfonyl, Carboxy, Niederalkoxycarbonyl, Carbamoyl, N-Niederalkylcarbamoyl, N,N-Diniederalkyl-carbamoyl oder Cyano substituiert ist.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist beispielsweise C₁-C₇-Alkyl, vorzugsweise C₁-C₄-Alkyl, wie insbesondere Methyl oder in zweiter Linie Äthyl, n-Propyl, Isopropyl oder n-Butyl, kann aber z.B. auch Isobutyl, sek.-Butyl, tert.-Butyl oder eine Pentyl-, Hexyl- oder Heptylgruppe sein.

Halogen-niederalkyl ist z.B. Trifluormethyl.

Niederalkenoyl ist z.B. C₃-C₇-Alkenoyl, vorzugsweise C₃-C₅-Alkenoyl, wie Prop-2-enoyl (Acryloyl), 2-Methylprop-2-enoyl (Methacryloyl), But-2-enoyl, But-3-enoyl 3-Methylbut-3-enoyl oder Pent-4-enoyl.

Halogen ist insbesondere Chlor oder Fluor, ferner Brom, kann aber auch für Iod stehen.

Halogen-niederalkanoyl ist beispielsweise Halogen-C₂-C₇-alkanoyl, wobei Halogen insbesondere Chlor bedeutet, aber in zweiter Linie auch Fluor oder Brom sein kann, vorzugsweise Chlor-C₂-C₄-alkanoyl, wie Chloracetyl, 3-Chlorpropionyl oder 4-Chlorbutyryl.

Diniederalkylamino-niederalkanoyl ist z.B. Di-C₁-C₄-Alkylamino-C₂-C₇-alkanoyl, und vorzugsweise N,N-Dimethylamino-C₂-C₄-alkanoyl, wie Dimethylaminoacetyl.

4- bis 6-Gliedriges Niederalkylenamino-niederalkanoyl ist z.B. Pyrrolidino- und vorzugsweise Piperidino-C₂-C₄-alkanoyl, etwa Piperidinoacetyl.

Niederalkoxy-niederalkyl trägt die Niederalkoxygruppe vorzugsweise in höherer als der α-Stellung und ist beispielsweise entsprechendes C₁-C₄-Alkoxy-C₂-C₄-alkyl, wie 2-Methoxyäthyl, 2-Äthoxyäthyl, 2-Propyloxyäthyl, 2-Isopropyloxyäthyl, 3-Methoxy-propyl, 3-Äthoxypropyl, 3-Isopropyloxypropyl oder 4-Methoxybutyl.

Carboxy-carbonyl steht für die Gruppe -C(=O)-COOH.

Aryl ist z.B. Phenyl, das unsubstituiert oder durch einen oder zwei Substituenten aus der Gruppe bestehend aus Niederalkyl, Niederalkoxy, Hydroxy, Halogen und Trifluormethyl substituiert ist, und vor allen Dingen Phenyl.

Acyl ist z.B. Niederalkanoyl.

Salze von Verbindungen der Formel I sind insbesondere pharmazeutisch verwendbare Salze, z.B. Säureadditionssalze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate), oder Salze mit starken organischen Carbonsäuren, wie Niederalkancarbonsäuren oder gegebenenfalls ungesättigten oder hydroxylierten aliphatischen Dicarbonsäuren, z.B. Acetate, Oxalate, Malonate, Maleinate, Fumarate, Maleate, Tartrate oder Citronate.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Verbindungen der Formel I und ihre pharmazeutisch verwendbaren Salze weisen wertvolle pharmakologische Eigenschaften auf. Insbesondere zeigen sie ausgeprägte Hemmwirkung auf die Biosynthese von Interleukin-1 (IL-1).IL-1 gehört zur Klasse der proinflammatorischen Proteine und spielt beispielsweise bei der Prostaglandinsynthese, der Synthese neutraler Proteasen durch Fibroplasten, Synovialzellen und Chondrozyten, bei der Aktivierung von Endothelialzellen und bei der Induktion weiterer proinflammatorischer Zytokine, wie des α-Tumornekrosefaktors (TNF) und von Interleukin-6 (IL-6) eine wesentliche Rolle. Ferner regt es die knochenresorption an, reguliert die Körpertemperatur von Warmblütern und reguliert u.a. die Entwicklung, Aktivierung, Differenzierung und Proliferation von Lymphozyten. Therapeutisch besonders wichtig ist die Hemmwirkung der Verbindungen der Formel I und ihrer pharmazeutisch verwendbaren Salze auf die Biosynthese von IL-1, TNF und 1L-6. Diese kann in vitro beispielsweise an durch Lipopolysaccharide stimulierten (LPS-stimulierten) menschlichen Monozyten nach C. Rordorf-Adam et al., Drugs Exptl. Clin. Res. XV, 355-62 (1989) im Konzentrationsbereich ab etwa 1 µMol und in vivo in der Maus anhand der Hemmung der LPS-induzierten Bildung von Serumamyloid P (SAP) bei einer ED₅₀ von etwa 1 bis 15 mg/kg p.o. und an der Ratte anhand der Senkung des durch LPS erzeugten künstlichen Fiebers bei einer ED₅₀ von etwa 0,05 bis 3,5 mg/kg p.o. gezeigt werden.

Auf Grund dieser Eigenschaften sind die Verbindungen der Formel I und ihre pharmazeutisch verwendbaren Salze vorzüglich geeignet zur therapeutischen Behandlung von Erkrankungen, bei denen eine übermässige Produktion von IL-1 eine ursächliche oder verschlimmernde Rolle spielt, wie entzündliche und degenerative Gelenkerkrankungen, beispielsweise der rheumatoiden Arthritis, Osteoarthrosis, psoriatischen oder infektösen Arthritis, des Reitersyndroms, von Gicht und traumatischer Arthritis, und anderer akuter oder chronischer Entzündungen, beispielsweise von entzündlichen Darmerkrankungen, von Meningitis, Hauterkrankungen, wie Psoriasis, Pemphigus vulgaris und dergl., von allergischen Hautreaktionen, Atherosklerose und Autoimmunerkrankungen, wie Diabetes (Typ 1) und Thyroiditis.

Als weitere Erkrankungen, bei denen eine übermässige Produktion von IL-1 eine ursächliche oder verschlimmernde Rolle spielt, sind z.B. zu nennen: Knochenmetabolismus-Regulationsstörungen, z.B. Paget-Krankheit, Osteoporose, Periodontitis oder Malignitäten; oder endotoxischer Schock, z.B. verbunden mit Fieber, Hypotension und fulminantem Leberversagen.

Zusätzlich weisen die Verbindungen der Formel I und ihre pharmazeutisch verwendbaren Salze eine ausgeprägte analgetische Wirkung auf, die sich beispielsweise anhand der Hemmung des durch Phenyl-p-benzochinon ausgelösten Writhingsyndroms der Maus beispielsweise in einer an Hendershot und Forsaith, J. Pharmacol. Exp. Therap. 125,237 (1959) angelehnten Versuchanordnung mit einer ED₅₀ von etwa 1 bis 30 mg/kg p.o. nachweisen lässt.

Die Verbindungen der Formel I und ihre pharmazeutisch verwendbaren Salze können dementsprechend auch als analgetische Arzneimittelwirkstoffe zur Behandlung von Schmerzzuständen unterschiedlicher Genese, insbesondere als periphere Analgetika, verwendet werden.

Bevorzugt betrifft die Erfindung die Verbindungen der Formel I, worin R₁ für Wasserstoff, Niederalkyl, Niederalkoxy-niederalkyl, Niederalkoxy-niederalkoxyniederalkyl; Phenyloxy-niederalkyl oder Phenylniederalkoxy-niederalkyl, wobei in den beiden letztgenannten Resten die Phenylgruppe jeweils unsubstituiert oder durch Niederalkyl, Trifluormethyl, Niederalkoxy, Halogen und/oder Hydroxy substituiert ist; N-Niederalkylamino-niederalkyl oder N,N-Diniederalkylamino-niederalkyl steht, R₂ Niederalkenoyl, (Carboxy oder Niederalkoxycarbonyl)-niederalkenoyl, Halogen-niederalkanoyl, Amino-niederalkanoyl, N-Niederalkylamino-niederalkanoyl, N-[(Carboxy oder Niederalkoxycarbonyl)-niederalkylamino]-niederalkanoyl, N,N-Diniederalkylamino-niederalkanoyl, C₄-C₆-Niederalkylenamino-niederalkanoyl, Morpholino-niederalkanoyl, Thiomorpholino-niederalkanoyl; Piperazino-niederalkanoyl, wobei der Piperazinorest unsubstituiert oder in 4-Stellung durch Niederalkyl oder Niederalkanoyl substituiert ist; Hydroxy-niederalkanoyl, Niederalkoxy-niederalkanoyl, Niederalkanoyloxy-niederalkanoyl, Niederalkylthio-niederalkanoyl, Niederalkylsulfinyl-niederalkanoyl, Niederalkylsulfonyl-niederalkanoyl; Phenylthio-niederalkanoyl, Phenylsulfinyl-niederalkanoyl oder Phenylsulfonyl-niederalkanoyl, wobei in den drei letztgenannten Resten die Phenylgruppe jeweils unsubstituiert oder durch Niederalkyl, Niederalkoxy, Halogen und/oder Hydroxy substituiert ist; Carboxy-niederalkanoyl, Niederalkoxycarbonyl-niederalkanoyl, Carbamoyl-niederalkanoyl, N-Niederalkylcarbamoyl-niederalkanoyl, N,N-Diniederalkylcarbamoyl-niederalkanoyl, Cyano-niederalkanoyl, Carbamoyl, N-Niederalkylcarbamoyl, N,N-Diniederalkylcarbamoyl, Carboxy-carbonyl, Niederalkoxycarbonyl-carbonyl, (Carbamoyl, N-Niederalkylcarbamoyl oder N,N-Diniederalkylcarbamoyl)-carbonyl bedeutet oder R₂, sofern R₁ für Niederalkoxyniederalkyl, Niederalkoxy-niederalkoxy-niederalkyl, unsubstituiertes oder wie oben definiert substituiertes Phenyloxy-niederalkyl oder Phenylniederalkoxy-niederalkyl, N-Niederalkylamino-niederalkyl oder N,N-Diniederalkylamino-niederalkyl steht, auch Wasserstoff, Niederalkanoyl, Carboxy, Niederalkoxycarbonyl oder Phenylniederalkoxycarbonyl, worin die Phenylgruppe unsubstituiert oder durch Niederalkyl, Niederalkoxy, Halogen und/oder Hydroxy substituiert ist, bedeuten kann, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, und X und Y unabhängig voneinander eine direkte Bindung, Niederalkylen oder Niederalkenylen bedeuten, und Salze davon.

Besonders bevorzugt betrifft die Erfindung die Verbindungen der Formel I, worin R₁ für Wasserstoff, Niederalkyl, Niederalkoxy-niederalkyl, Niederalkoxy-niederalkoxyniederalkyl, Phenyloxy-niederalkyl, N-Niederalkylamino-niederalkyl oder N,N-Diniederalkylamino-niederalkyl steht, R₂ Niederalkenoyl, (Carboxy oder Niederalkoxycarbonyl)-niederalkenoyl, Halogen-niederalkanoyl, N-Niederalkylamino-niederalkanoyl, N-[(Carboxy oder Niederalkoxycarbonyl)-niederalkylamino]-niederalkanoyl, N,N-Diniederalkylamino-niederalkanoyl, Piperidino-niederalkanoyl, Hydroxy-niederalkanoyl, Niederalkoxy-niederalkanoyl, Niederalkanoyloxy-niederalkanoyl, Niederalkylthio-niederalkanoyl, Niederalkylsulfinyl-niederalkanoyl, Niederalkylsulfonyl-niederalkanoyl, Phenylthio-niederalkanoyl, Phenylsulfinyl-niederalkanoyl, Phenylsulfonyl-niederalkanoyl, Carboxy-niederalkanoyl, Niederalkoxycarbonyl-niederalkanoyl, N,N-Diniederalkylcarbamoyl oder Niederalkoxycarbonyl-carbonyl bedeutet oder R₂, sofern R₁ für Niederalkoxy-niederalkyl oder N,N-Diniederalkylamino-niederalkyl steht, auch Wasserstoff, Niederalkanoyl, Niederalkoxycarbonyl oder Phenylniederalkoxycarbonyl bedeuten kann, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Trifluormethyl, Niederalkoxy, Niederalkylthio, Halogen oder Diniederalkylamino stehen, und X und Y unabhängig voneinander eine direkte Bindung, Niederalkylen oder Niederalkenylen bedeuten, und Salze davon.

Ganz besonders bevorzugt betrifft die Erfindung die Verbindungen der Formel I, worin R₁ für Wasserstoff, Niederalkyl oder Niederalkoxy-niederalkyl steht, R₂ Niederalkenoyl oder Halogen-niederalkanoyl bedeutet oder R₂, sofern R₁ für Niederalkoxy-niederalkyl steht, auch Wasserstoff bedeuten kann, R₃ und R₄ Wasserstoff bedeuten, R₅ für Chlor steht, R₆ Wasserstoff bedeutet, X für eine direkte Bindung steht und Y 1,2-Aethylen bedeutet, und pharmazeutisch verwendbare Salze davon.

Die Erfindung betrifft ferner insbesondere die Verbindungen der Formel Ia, 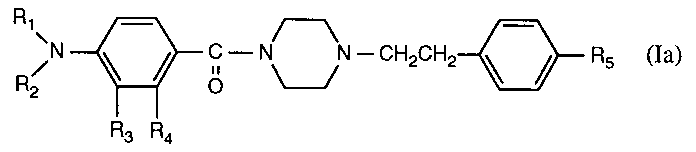
worin R₁ für Wasserstoff, Niederalkyl oder Niederalkoxyniederalkyl steht, R₂ Niederalkenoyl oder durch Halogen, Diniederalkylamino, 4- bis 6-gliedriges Niederalkylenamino oder Niederalkylthio substituiertes Niederalkanoyl bedeutet oder R₂, sofern R₁ Niederalkoxyniederalkyl darstellt, Wasserstoff bedeuten kann, R₃ und R₄ unabhängig voneinander Wasserstoff, Niederakyl, Chlor, Fluor oder Brom bedeuten und R₅ Niederalkylthio, Chlor, Fluor oder Brom darstellt, und Salze davon.

Die Erfindung betrifft in erster Linie Verbindungen der Formel Ia, worin R₁ Wasserstoff, Niederalkyl oder Niederalkoxyniederalkyl bedeutet, R₂ für durch Halogen, Niederalkylthio, Niederalkylsulfinyl oder Niederalkylsulfonyl substituiertes Niederalkanoyl oder, sofern R₁ Niederalkoxyniederalkyl bedeutet, für Wasserstoff stehen kann, R₃ und R₄ unabhängig voneinander Wasserstoff, Niederalkyl, Chlor, Fluor oder Brom bedeuten und R₅ Niederalkylthio, Chlor, Fluor oder Brom darstellt, und ihre Salze.

Die Erfindung betrifft vor allem Verbindungen der Formel Ia, worin R₁ Wasserstoff, C₁-C₄-Alkyl oder C₁-C₄-Alkoxy-C₂-C₄-alkyl bedeutet, R₂ C₃-C₇-Alkenoyl, Chlor-C₂-C₄-alkanoyl, C₁-C₄-Alkylthio-, C₁-C₄-Alkylsulfinyl- oder C₁-C₄-Alkylsulfonyl-C₂-C₄-alkanoyl oder, sofern R₁ C₁-C₄-Alkoxy-C₂-C₄-alkyl bedeutet, Wasserstoff darstellen kann, R₃ und R₄ unabhängig voneinander Wasserstoff, C₁-C₄-Alkyl, Fluor oder Chlor bedeuten und R₅ für Chlor steht, und pharmazeutisch verwendbare Salze davon.

Die Erfindung betrifft insbesondere Verbindungen der Formel I, worin R₁ Wasserstoff, Methyl, Äthyl oder 2-Isopropyloxyäthyl bedeutet, R₂ Prop-2-enoyl, Chloracetyl, 3-Chlorpropionyl, Methylthioacetyl oder Äthylthioacetyl darstellt, R₃ und R₄ Wasserstoff, Methyl, Fluor oder Chlor bedeuten und R₅ für Chlor steht, und pharmazeutisch verwendbare Salze davon.

Die Erfindung betrifft namentlich die in den Beispielen genannten Verbindungen der Formel I und ihre Salze, insbesondere ihre pharmazeutisch verwendbaren Salze.

Das Verfahren zur Herstellung der Verbindungen der Formel I beruht auf an sich bekannten Methoden und ist z.B. dadurch gekennzeichnet, dass man
a) eine Verbindung der Formel II, 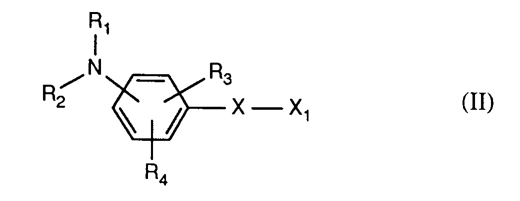 worin X₁ Carboxy oder reaktionsfähiges funktionell abgewandeltes Carboxy bedeutet, oder ein Salz davon, mit einer Verbindung der Formel III, 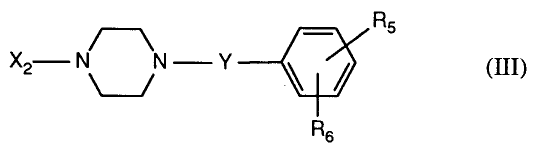 worin X₂ Wasserstoff oder eine Aminoschutzgruppe bedeutet, umsetzt, oder
b) eine Verbindung der Formel IV, 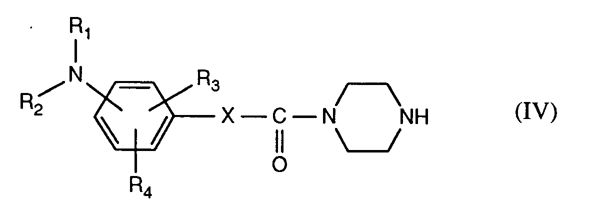 oder ein Salz davon, mit einer Verbindung der Formel V, 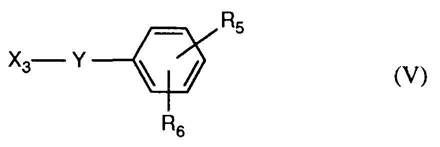 worin X₃ Hydroxy oder reaktionsfähiges verestertes Hydroxy bedeutet, umsetzt, oder
c) eine Verbindung der Formel VI, 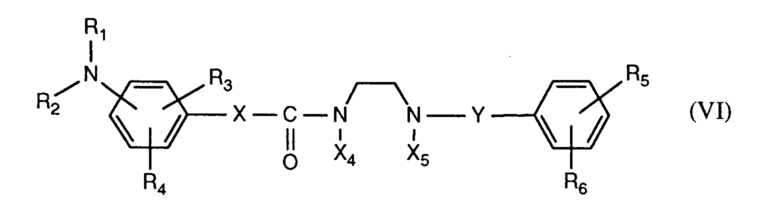 worin einer der Reste X₄ und X₅ Wasserstoff und der andere eine Gruppe der Formel -CH₂-CH₂-X₃ (VIa) bedeutet und X₃ für Hydroxy oder reaktionsfähiges verestertes Hydroxy steht, cyclisiert, oder
d) eine Verbindung der Formel VII, 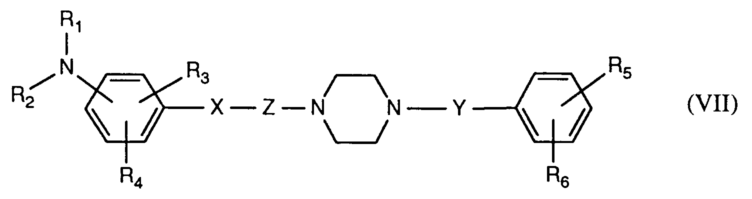 worin Z eine zu Carbonyl oxidierbare Gruppe bedeutet, oxidiert, oder
e) in eine Verbindung der Formel VIII, 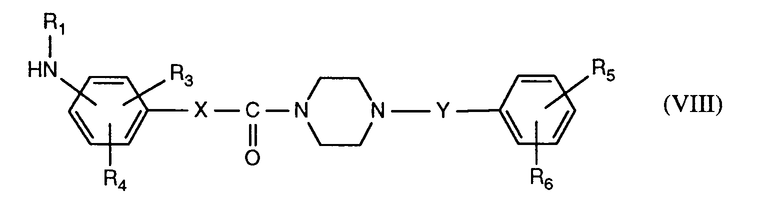 den Rest R₂ einführt (R₂ ≠ H), oder
f) zur Herstellung von Verbindungen, worin R₁ Niederalkoxyniederalkyl bedeutet, in eine Verbindung der Formel IX

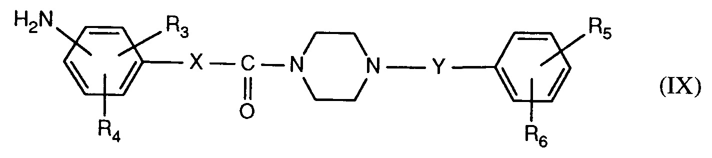

den Rest R₁ einführt; und jeweils gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensgemäss erhältliches Salz in die freie Verbindung der Formel I oder in ein anderes Salz überführt.

Die vor- und nachstehend beschriebenen Umsetzungen werden in an sich bekannter Weise durchgeführt, z.B. in Ab- oder üblicher Weise in Anwesenheit eines geeigneten Lösungs-oder Verdünnungsmittels oder eines Gemisches derselben, wobei man je nach Bedarf unter Kühlen, bei Raumtemperatur oder unter Erwärmen, z.B. in einem Temperaturbereich von etwa -78° bis zur Siedetemperatur des Reaktionsmediums, vorzugsweise von etwa -10° bis etwa 150°C, und, falls erforderlich, in einem geschlossenen Gefäss, unter Druck, in einer Inertgasatmosphäre und/oder unter wasserfreien Bedingungen arbeitet.

In den Ausgangsmaterialien kann das basische Zentrum z.B. in Form von Säureadditionssalzen, beispielsweise mit den vorstehend im Zusammenhang mit Salzen von Verbindungen der Formel I aufgeführten Säuren, vorliegen, während Ausgangsverbindungen der Formel II, worin X₁ Carboxy bedeutet, Salze mit Basen bilden können. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Übergangsmetallsalze, wie Zink- oder Kupfersalze, oder Salze mit Ammoniak oder organischen Aminen, wie cyclische Amine, wie Mono-, Di- bzw. Tri-hydroxy-C₁-C₇-alkylamine, Hydroxy-C₁-C₇-alkyl-C₁-C₇-alkyl-amine oder wie Polyhydroxy-C₄-C₇-alkylamine. Cyclische Amine sind z.B. Morpholin, Thiomorpholin, Piperidin oder Pyrrolidin. Als Mono-C₁-C₇-alkylamine kommen beispielsweise Äthyl-oder tert.- Butylamin, als Di-C₁-C₇-alkylamine beispielsweise Diäthyl- oder Diisopropylamin, als Tri-C₁-C₇-alkylamine beispielsweise Trimethyl- oder Triäthylamin in Betracht. Entsprechende Hydroxy-C₁-C₇-alkylamine sind z.B. Mono-, Di- bzw. Triäthanolamine, und Hydroxy-C₁-C₇-alkyl-C₁-C₇-alkylamine sind z.B. N,N-Dimethylamino- oder N,N-Diäthylaminoäthanol, ferner Glucosamin als Polyhydroxy-C₆-alkylamin.

Reaktionsfähiges funktionell abgewandeltes Carboxy X₁ bedeutet beispielsweise verestertes, in erster Linie reaktionsfähiges verestertes, Carboxy, anhydridisiertes Carboxy oder amidiertes Carboxy.

Verestertes Carboxy ist beispielsweise gegebenenfalls substituiertes C₁-C₇-Alkoxy-carbonyl, wie Äthoxycarbonyl, vorzugsweise jedoch reaktionsfähiges verestertes Carboxy, beispielsweise gegebenenfalls, z.B. durch C₁-C₇-Alkoxy oder gegebenenfalls substituiertes Carbamoyl, zusätzlich aktiviertes Vinyloxycarbonyl, wie 1-C₁-C₇-Alkoxy-, z.B. 1-Äthoxyvinyloxycarbonyl, oder 2-(N-C₁-C₇-Alkyl-carbamoyl)-, z.B. 2-(N-Äthyl-carbamoyl)-vinyloxycarbonyl, sowie gegebenenfalls, z.B. durch Nitro, Halogen, C₁-C₇-Alkansulfonyl oder Phenylazo, substituiertes Phenoxy- bzw. Thiophenoxycarbonyl, wie 4-Nitro-, 2,4,5-Trichlor-, Pentachlor-, 4-Methansulfonyl-, 4-Phenylazo-phenoxy-carbonyl, Thiophenoxy- oder 4-Nitrothiophenoxycarbonyl, und ebenso aktiviertes, z.B. durch Cyano oder ferner gegebenenfalls verestertes Carboxy substituiertes, Methoxycarbonyl, insbesondere Cyanomethoxycarbonyl. Reaktionsfähiges verestertes Carboxy kann ebenso 1,1- oder 1,3-disubstituiertes 2-Isoureidocarbonyl, wie 1,1-Diniederalkyl-, 1,1-Diaryl- oder 1,1-Diaryl-C₁-C₇-alkyl-2-isoureidocarbonyl, z.B. 1,1-Diäthyl-, 1,1-Diphenyl- oder 1,1-Dibenzyl-2-isoureidocarbonyl, oder 1,3-Dicycloalkyl-, z.B. 1,3-Dicyclohexyl-2-isoureido-carbonyl, oder N-C₂-C₇-Alkylenaminooxycarbonyl, wie N-Piperidinyl-oxycarbonyl, sowie N-Imido-oxycarbonyl, z.B. N-Succinimido-oxy- oder N-Phthalimido-oxycarbonyl, sein.

Unter anhydridisiertem Carboxy ist beispielsweise gegebenenfalls verzweigtes C₁-C₇-Alkoxycarbonyloxycarbonyl, wie Äthoxy- oder Isobutyloxycarbonyloxycarbonyl, Halogencarbonyl, wie Chlorcarbonyl, Azidocarbonyl, Halogenphosphoryloxycarbonyl, wie Dichlorphosphoryloxycarbonyl, oder gegebenenfalls, z.B. durch Halogen oder Aryl, substituiertes C₁-C₇-Alkanoyloxycarbonyl, wie Pivaloyloxy-, Trifluoracetyloxy- oder Phenylacetoxycarbonyl, zu verstehen.

Reaktionsfähiges amidiertes Carboxy ist beispielsweise gegebenenfalls, z.B. durch C₁-C₇-Alkyl, substituiertes 1-Imidazolyl- oder 1-Pyrazolylcarbonyl, wie 3,5-Dimethyl-pyrazolylcarbonyl.

Eine Aminoschutzgruppe X₂ ist beispielsweise Acyl, wie C₁-C₇-Alkanoyl, z.B. Formyl oder Acetyl, Halogencarbonyl, wie Chlorcarbonyl, ferner gegebenenfalls substituiertes Aryl- oder Heteroarylsulfonyl, wie 2-Pyridyl- oder 2-Nitrophenylsulfonyl.

Im Rahmen der vor- und nachstehenden Verfahrensbeschreibung bedeutet reaktionsfähiges verestertes Hydroxy, z.B. X₃, sofern nicht abweichend definiert, insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes C₁-C₇-Alkansulfonyloxy, z.B. Methan- oder Trifluor-methansulfonyloxy, C₃-C₇-Cycloalkansulfonyloxy, z.B. Cyclohexansulfonyloxy, oder gegebenenfalls, z.B. durch C₁-C₇-Alkyl oder Halogen, substituiertes Benzolsulfonyloxy, z.B. p-Bromphenyl- oder p-Toluolsulfonyloxy.

Werden bei den vor- und nachstehend beschriebenen Umsetzungen beispielsweise Basen verwendet, so kommen, sofern nicht abweichend aufgeführt, beispielsweise Alkalimetallhydroxide, -hydride, -amide, -alkanolate, -carbonate, -triphenylmethylide, -di-C₁-C₇-alkylamide, -amino-C₁-C₇-alkylamide oder -C₁-C₇-alkylsilylamide, Naphthyl-amine, C₁-C₇-Alkylamine, basische Heterocyclen, Ammoniumhydroxide sowie carbocyclische Amine in Frage. Beispielhaft seien Lithiumhydroxid, Natriumhydroxid, -hydrid, -amid, -äthylat, Kaliumtertiärbutanolat, -carbonat, Lithiumtriphenylmethylid, -diisopropylamid, Kalium-3-(aminopropyl)-amid, -bis-(trimethylsilyl)-amid, Dimethylaminonaphthalin, Di- oder Triäthylamin, Pyridin, Benzyl-trimethylammoniumhydroxid, 1,5-Diaza-bicyclo[4.3.0]non-5-en (DBN) sowie 1,8-Diaza-bicyclo[5.4.0]undec-7-en (DBU) genannt.

### Variante a):

Die verfahrensgemässe N-Acylierung wird in an sich bekannter Weise durchgeführt, erforderlichenfalls in Gegenwart eines, insbesondere basischen, Kondensationsmittels. Als Basen kommen beispielsweise Vertreter der vorstehend aufgeführten Basen in Frage. Häufig ist auch die Basizität der Verbindung der Formel III ausreichend.

Falls X₁ Carboxy bedeutet, werden z.B. primär die entsprechenden Ammoniumsalze gebildet, die durch Erwärmen oder Behandeln mit geeigneten Dehydratisierungsmitteln (als Kondensationsmittel), wie Carbodiimiden, z.B. N,N′-Diniederalkyl- oder N,N′-Dicycloalkylcarbodiimid, wie N,N′-Diäthyl-, N,N′-Diisopropyl- oder N,N′-Di-cyclohexyl-carbodiimid, vorteilhaft unter Zusatz von N-Hydroxysuccinimid oder gegebenenfalls, z.B. durch Halogen, Niederalkoxy oder Niederalkyl, substituiertem 1-Hydroxy-benzotriazol oder N-Hydroxy-5-norbornen-2,3-dicarboxamid, sowie N,N-Carbonyldiimidazol, dehydratisiert werden können. Mit Carbodiimiden können intermediär z.B. auch die entsprechenden 1-Isoureidocarbonyl-Verbindungen gebildet werden. Als wasserbindende Kondensationsmittel können weiterhin N-Äthoxycarbonyl-2-ethoxy-1,2-dihydrochinolin, Phosphorylcyanamide bzw. -azide, wie Diäthylphosphoryl-cyanamid oder Diphenylphosphorylazid, Triphenylphosphin-disulfid oder 1 -Niederalkyl-2-halogeno-piperidinium-halogenide, wie 1-Methyl-2-chlor- pyridinium-iodid, eingesetzt werden.

Die in dieser Verfahrensvariante verwendeten Ausgangsstoffe sind z.T. bekannt bzw können nach an sich bekannten Verfahren hergestellt werden.

Zur Herstellung von Verbindungen der Formel II, worin X₁ gegebenenfalls substituiertes C₁-C₇-Alkoxycarbonyl bedeutet, kann man üblicherweise von der freien Säure (X₁ = Carboxy) oder einem Säureanhydrid (X₁ bedeutet z.B. Halogencarbonyl) ausgehen und diese beispielsweise mit dem entsprechenden, erforderlichenfalls in reaktionsfähiger Form vorliegenden, Alkohol, z.B. einem C₁-C₇-Alkylhalogenid, umsetzen. Die Herstellung von Verbindungen der Formel II, worin X₁ gegebenenfalls zusätzlich aktiviertes Vinyloxycarbonyl bedeutet, kann z.B. durch Umesterung eines C₁-C₇-Alkylesters mit Vinylacetat (Methode des aktivierten Vinylesters), durch Umsetzung der freien Säure von Verbindungen der Formel II mit Niederalkoxyacetylen (z.B. Äthoxyacetylen-Methode) oder, analog der Woodward-Methode, mit einem 1,2-Oxazoliumsalz erfolgen. Gegebenenfalls substituiertes Phenoxy- bzw. Thiophenoxycarbonyl aufweisende Verbindungen der Formel II können beispielsweise ausgehend von der freien Säure nach der Carbodiimid-Methode durch Umsetzen mit dem entsprechenden (Thio-)Phenol durchgeführt werden. Ebenfalls ausgehend von der freien Säure der Formel II können Verbindungen der Formel II, worin X₁ aktiviertes Methoxycarbonyl bzw. 1,1- oder 1,3-disubstituiertes 2-Isoureidocarbonyl darstellt, z.B. durch Umsetzung mit einem Halogen-, wie Chloracetonitril (Cyanmäthylester-Methode) bzw. mit einem Carbodiimid oder Cyanamid (Carbodiimid- oder Cyamid-Methode) erhalten werden. Die Herstellung von N-C₂-C₇-Alkylenamino-oxycarbonyl- bzw. N-Imido-oxycarbonyl-Verbindungen der Formel II kann beispielsweise bei Verwendung der freien Säure der Formel II aus entsprechenden N-Hydroxyverbindungen mit Hilfe von Carbodiimiden nach der Methode der aktivierten N-Hydroxyester erfolgen. Zur Herstellung von Verbindungen der Formel II, worin X₁ gegebenenfalls verzweigtes C₁-C₇-Alkoxycarbonyloxycarbonyl, Halogenphosphoryloxy-carbonyl bzw. gegebenenfalls substituiertes C₁-C₇-Alkanoyloxycarbonyl bedeutet, kann beispielsweise von freier Säure der Formel II ausgegangen werden, die z.B. mit einem entsprechenden Halogenid, wie gegebenenfalls substituiertes C₁-C₇-Alkylkohlensäure-halogenid (Methode der gemischten O-Kohlensäureanhydride), Phosphoroxyhalogenid (z.B. Phosphoroxychlorid-Methode) oder gegebenenfalls substituiertes C₁-C₇-Alkanoyl-halogenid (Methode der gemischten Carbonsäurehalogenide) behandelt werden kann. Azidocarbonylverbindungen der Formel II sind beispielsweise durch Behandeln entsprechender Hydrazide mit salpetriger Säure zugänglich (Azid-Methode). Zur Herstellung von Verbindungen der Formel II, worin X₁ gegebenenfalls substituiertes 1-Imidazolyl-carbonyl bzw. 1-Pyrazolyl-carbonyl bedeutet, setzt man die freie Säure der Formel II z.B. mit Di-(1-imidazolyl)-carbonyl (Imidazolid-Methode) bzw. das betreffende Hydrazid z.B. mit einem entsprechenden 1,3-Diketon (Pyrazolid-Methode) um.

### Variante b):

Der Rest X₃ bedeutet insbesondere reaktionsfähiges verestertes Hydroxy, z.B. Halogen, wie Chlor.

Die verfahrensgemässe N-Alkylierung wird in an sich bekannter Weise durchgeführt, erforderlichenfalls in Gegenwart einer, z.B. der vorstehend aufgeführten, Basen.

Die in dieser Verfahrensvariante verwendeten Ausgangsstoffe sind z.T. bekannt oder können in an sich bekannter Weise hergestellt werden.

So kann beispielsweise das Ausgangsmaterial der Formel IV hergestellt werden, indem man eine Verbindung der Formel II oder ein Salz davon, worin X₁ Carboxy oder reaktionsfähiges funktionell abgewandeltes Carboxy bedeutet, mit einer Verbindung der Formel IVa 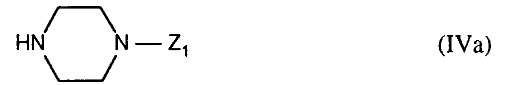
oder einem Salz davon, worin Z₁ Wasserstoff oder eine Aminoschutzgruppe, wie Benzyl, bedeutet, in der in Variante a) beschriebenen Weise umsetzt und gegebenenfalls die Aminoschutzgruppe, z.B. Benzyl durch übliche Hydrogenolyse, abspaltet.

### Variante c):

Die verfahrensgemässe Cyclisierung (intramolekulare N-Alkylierung) wird in an sich bekannter Weise, erforderlichenfalls in Gegenwart eines, insbesondere basischen, Kondensationsmittels durchgeführt. Als Basen werden z.B. vorstehend aufgeführten verwendet.

X₃ bedeutet dabei insbesondere reaktionsfähiges verestertes Hydroxy, bevorzugt Halogen, wie Chlor.

Das Ausgangsmaterial kann in an sich bekannter Weise hergestellt werden. Beispielsweise geht man von einer Verbindung der Formel II oder einem Salz davon aus, worin X₁ Carboxy oder reaktionsfähiges funktionell abgewandeltes Carboxy bedeutet, und setzt diese zunächst mit einer Verbindung der Formel 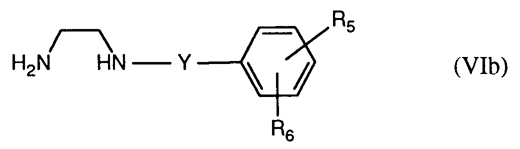
in Analogie zu Variante a) um. Im nächsten Reaktionsschritt wird die erhaltene Verbindung mit einer Verbindung der Formel X₃-CH₂-CH₂-X₃ (VIc) unter N-alkylierenden Bedingungen gemäss Variante b) umgesetzt.

### Variante d):

Eine zu -CO- oxidierbare Gruppe Z steht in erster Linie für -CH₂-. Die Oxidation entsprechender Verbindungen der Formel VII erfolgt mit Hilfe eines geeigneten Oxidationsmittels, wobei bevorzugt gegebenenfalls, z.B. durch einen Phenylrest, substituierte Tetra-C₁-C₄-alkylammoniumpermanganate, in erster Linie Benzyl-triäthylammoniumpermanganat, verwendet wird.

Das Ausgangsmaterial der Formel VII wird in an sich bekannter Weise hergestellt. Beispielsweise geht man von einer Verbindung der Formel III aus, worin X₂ Wasserstoff bedeutet, und setzt diese unter den in Variante b) beschriebenen N-alkylierenden Bedingungen mit einer Verbindung der Formel VIIa 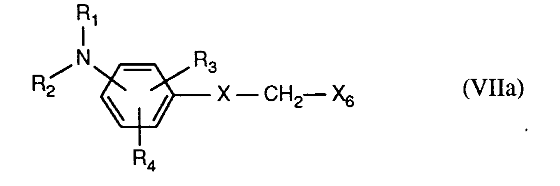
um, wobei X₆ Hydroxy oder in erster Linie reaktionsfähiges verestertes Hydroxy, insbesondere Halogen, wie Chlor oder Brom, bedeutet.

### Variante e):

Die Einführung von Niederalkenoyl bzw. elektronegativ substituiertem Niederalkanoyl (N-Acylierung) erfolgt in üblicher Weise, erforderlichenfalls in Gegenwart eines, insbesondere basischen, Kondensationsmittels. Als Basen kommen beispielsweise Vertreter der vorstehend aufgeführten Basen in Frage.

### Variante f):

Einführung von Niederalkoxyniederalkyl R₁ (N-Alkoxyalkylierung) erfolgt in üblicher Weise, erforderlichenfalls in Gegenwart eines, insbesondere basischen, Kondensationsmittels. Als Basen kommen beispielsweise Vertreter der vorstehend aufgeführten Basen in Frage.

Eine verfahrensgemäss oder anderweitig erhältliche erfindungsgemässe Verbindung kann in an sich bekannter Weise in eine andere erfindungsgemässe Verbindung übergeführt werden.

In erfindungsgemässen Verbindungen, in welchen R₁ Niederalkoxyniederalkyl und R₂ Wasserstoff ist, kann die Aminogruppe in der vorstehend unter Variante a), b) oder e) angegebenen Weise N-acyliert werden. Ebenso kann eine Verbindung der Formel I, worin R₁ Wasserstoff und R₂ Niederalkenoyl oder elektronegativ substituiertes Niederalkanoyl bedeutet, entsprechend der für die Verfahrensvariante f) angegebenen Weise N-niederalkyliert oder durch Niederalkoxyniederalkyl N-substituiert werden. Die N-Niederalkylierung kann dabei auch analog der Leuckart-Wallach- (bzw. Eschweiler-Clarke)-Reaktion aus Carbonylverbindungen, z.B. unter Verwendung von Ameisensäure als Reduktionsmittel, reduktiv durchgeführt werden.

Erhaltene Salze können in an sich bekannter Weise in die freien Verbindungen umgewandelt werden, z.B. durch Behandeln mit einer Base, wie einem Alkalimetall-hydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I z.B. in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgenden Beispiele dienen zur Illustration der Erfindung; Temperaturen sind in Celsiusgraden, Drucke in mbar angegeben. Folgende Abkürzungen werden verwendet: RT = Raumtemperatur, AT = Aussentemperatur, IT = Innentemperatur, Aether = Diäthyläther, Essigester = Aethylacetat = Essigsäureäthylester, (BOC₂)O = Di-tert.-Butyl-dicarbonat.

### Beispiel 1:

3.4 g 1-(4-Aminobenzoyl)-4-[2-(4-chlorphenyl)-äthyl]-piperazin und 2.6 g 2-Isopropyloxyäthyl-p-toluolsulfonat werden in 30 ml Toluol suspendiert und zum Sieden erhitzt. Die so erhaltene klare Lösung wird 17 Stunden am Rückfluss gekocht und anschliessend eingedampft. Der Rückstand wird mit Wasser versetzt, mit konzentrierter Natronlauge alkalisch gestellt und mit Methylenchlorid ausgeschüttelt. Das rohe Öl wird an Kieselgel chromatographiert und als Hydrochlorid aus Aceton kristallisiert. Es liegt das 1-{4-[N-(2-Isopropyloxyäthyl)-amino]-benzoyl}-4-[2-(4-chlorphenyl)-äthyl]-piperazin-hydrochlorid vom Smp. 184-185° vor.

Das Ausgangsmaterial wird wie folgt hergestellt:
37.2 g 1-(4-Nitrobenzoyl)-4-[2-(4-chlorphenyl)-äthyl]-piperazin werden in 370 ml Tetrahydrofuran in Anwesenheit von 20 g Raney-Nickel bei Raumtemperatur reduziert. Die filtrierte Reaktionslösung wird eingedampft und das erhaltene Öl aus Isopropanol/Petroläther kristallisiert. Man erhält so 30 g 1-(4-Aminobenzoyl)-4-[2-(4-chlorphenyl)-äthyl]-piperazin vom Smp. 111-112°.

Das 1-(4-Nitrobenzoyl)-4-[2-(4-chlorphenyl)-äthyl]-piperazin wird aus 4-Nitro-benzoesäure und 1-[(4-chlorphenyl)äthyl]-piperazin in N,N-Dimethylformamid in Anwesenheit von N,N-Carbonylimidazol hergestellt. Es schmilzt bei 109-110⁰.

### Beispiel 2:

69.8 g 1-{4-[N-(2-Isopropyloxyäthyl)-N-tertiärbutyloxycarbonyl-amino]-benzoyl}-4-[2-(4-chlorphenyl)-äthyl]-piperazin werden in 900 ml Methylenchlorid gelöst und mit 600 ml Trifluoressigsäure versetzt. Die dunkle Lösung wird über Nacht bei Raumtemperatur stehen gelassen und im Vakuum eingedampft. Der Rückstand wird mit eiskaltem Wasser versetzt, mit Ammoniak alkalisch gestellt und mit Methylenchlorid ausgeschüttelt. Das dunkle Öl wird an Kieselgel chromatographiert. Das Methylenchlorid/Aceton-Eluat (7:3) wird eingedampft und am Vakuum getrocknet. Das ölige 1-{4-[N-(2-Isopropyloxyäthyl)-amino]-benzoyl}-4-[2-(4-chlorphenyl)-äthyl]piperazin wird langsam fest; Smp. 61-63°.

Das Ausgangsmaterial wird wie folgt hergestellt: 38 g 4-(N-Acetylamino)-benzoyl-4-[2-(4-chlorphenyl)-äthyl]-piperazin (s. EP-A-250 361) werden in 400 ml Acetonitril gelöst. Dazu werden 1.2 g 4-Dimethylaminopyridin gelöst in 20 ml Acetonitril gegeben und danach 24.5 g BOC₂O in 100 ml Acetonitril zugetropft. Man rührt 6 Std. bei RT und gibt dann weitere 5 g (BOC₂)O hinzu. Nach 5 Min. bei 35° und einer Std. bei RT werden 20 g 2-Diäthylaminoäthylamin zugegeben. Es wird über Nacht bei RT gerührt, wobei das intermediär gebildete 1-{4-[N-(Acetyl)-N-tertiärbutyloxycarbonyl-amino]-benzoyl}-4-[2-(4-chlorphenyl)-äthyl]-piperazin zu 1-[N-(tert.-Butyloxycarbonyl-amino)-benzoyl]-4-[2-(4-chlorphenyl)-äthyl]-piperazin gespalten wird. Es wird isoliert durch Eindampfen, Extrahieren mit Äthylacetat und Kristallisation aus Äther. Es schmilzt bei 129-131⁰.

4.4 g 1-[N-(tert.-Butyloxycarbonyl-amino)-benzoyl]-4-[2-(4-chlorphenyl)-äthyl]-piperazin werden in 15 ml DMSO gelöst. Man gibt 0.67 g pulv. KOH dazu. Man rührt 15 Min. bei RT und tropft anschliessend innerhalb von 5 Min. eine Lösung von 3 g O-(2-Isopropyloxyäthyl)-tosylat in 10 ml DMSO hinzu. Man rührt 7 Std. bei RT und 3 Std. bei 60-70⁰. Die Lösung wird bei RT über Nacht stehen gelassen. Sie wird auf 150 ml Wasser gegossen, abdekantiert und nochmals auf 150 ml Wasser gegossen. Das ausgeschiedene Öl wird mit Äthylacetat extrahiert, mit Wasser neutral gewaschen und eingedampft. Man erhält so das 1-{4-[N-(2-Isopropyloxyäthyl)-N-tertiärbutyloxy-carbonyl-amino]-benzoyl}-4-[2-(4-chlorphenyl)-äthyl]-piperazin vom Smp. 182-184⁰.

### Beispiel 3:

22 g 1-{4-[N-(2-Isopropyloxyäthyl)-amino]-benzoyl}-4-[2-(4-chlorphenyl)-äthyl]-piperazin (Beispiel 2) und 20.7 g Pottasche werden in 650 ml Toluol vorgelegt und 15 Minuten bei 45° gehalten. Bei dieser Temperatur und unter Rühren wird eine Lösung von 7.1 g Chloracetylchlorid in 30 ml Toluol zugetropft. Man rührt 30 Minuten bei 45° und danach bei Raumtemperatur weiter. Die Reaktionslösung wird mit 500 ml Essigester verdünnt und zweimal mit je 250 ml Wasser gewaschen. Das nach dem Eindampfen erhaltene Öl wird an Kieselgel chromatographiert und als Hydrochlorid aus Aceton-Äther umkristallisiert. Man erhält so das 1-{4-[N-(2-Isopropyloxyäthyl)-N-chloracetyl-amino]-benzoyl}-4-[2-(4-chlorphenyl)-äthyl]-piperazin-hydrochlorid vom Smp. 146-147°.

Nach Behandlung des Hydrochlorids mit Ammoniak erhält man die freie Base, die mit Dichlormethan extrahiert wird. Es wird eingedampft und an Kieselgel chromatographiert (Eluiermittel: Essigester). Die ölige Base wird aus Isopropyläther kristallisiert; Smp. 83-84°.

Zu einer Lösung von 4.83 g der freien Base in 50 ml Äthanol gibt man 1.06 g Fumarsäure in 70 ml Äthanol; nach 3 Tagen bei RT deutet sich eine Kristallisation an. Nach Zugabe von etwas Aceton und Äther und weiteren 2 Tagen Stehenlassen bei RT und schliesslich 12 Std. bei 0⁰ erhält man eine Suspension, die abgenutscht wird. Der Rückstand wird über KOH im Trockenschrank bei 60⁰ getrocknet. Man erhält das 1-{4-[N-(2-Isopropyloxyäthyl)-N-chloracetyl-amino]-benzoyl}-4-[2-(4-chlorphenyl)-äthyl]-piperazin-fumarat vom Smp. 105-107⁰.

### Beispiel 4:

In analoger Weise wie in den Beispielen 1 bis 3 beschrieben kann man ferner herstellen:
(a) 1-[4-(N-Methyl-N-chloracetyl-amino)-benzoyl]-4-[2-(4-chlorphenyl)-äthyl]-piperazin; Smp. 113-115°.
(b) 1-{4-[N-(3-Methylbutyl)-N-chloracetyl-amino]-benzoyl}-4-[2-(4-chlorphenyl)-äthyl]-piperazin;
(c) 1-[2-Chlor-4-(N-chloracetylamino)-benzoyl]-4-[2-(4-chlorphenyl)-äthyl]-piperazin;
(d) 1-[3-Methoxy-4-(N-chloracetylamino)-benzoyl]-4-[2-(4-chlorphenyl)-äthyl]-piperazin
(e) 1-{4-[N-(4-Methylpentyl)-N-chloracetyl-amino]-benzoyl}-4-[2-(4-chlorphenyl)äthyl]-piperazin; Smp. 90-91°.

### Beispiel 5:

11 g 1-(4-Aminobenzoyl)-4-[2-(4-chlorphenyl)-äthyl]-piperazin und 5.7 g 3-Äthoxypropylbromid werden in 150 ml Isopropanol suspendiert und bei 100⁰ in Lösung gebracht. Man gibt 9.4 g Pottasche dazu und man kocht 20 Stunden am Rückfluss. Nach dem Eindampfen und Chromatographieren an Kieselgel erhält man ein Oel, das aus Methylenchlorid und Petroläther kristallisiert wird. Man erhält so das 1-[4-(3-Äthoxy-propylamino)-benzoyl]-4-[2-(4-chlorphenyl)-äthyl]-piperazin vom Smp. 87-89°.

### Beispiel 6:

1.7 g 1-(4-Aminobenzoyl)-4-[2-(4-chlorphenyl)-äthyl]-piperazin und 0.55 g Triäthylamin werden in 25 ml Methylenchlorid vorgelegt. Dazu wird eine Lösung von 0.6 g Chloracetylchlorid zugetropft. Die dunkle Lösung wird über Nacht stehen gelassen, mit 50 ml Methylenchlorid verdünnt, mit 1 N Natronlauge gewaschen, über Magnesiumsulfat getrocknet und eingedampft. Das so erhaltene Kristallisat wird aus Aceton umkristallisiert. Es liegt das 1-(4-Chloracetylaminobenzoyl)-4-[2-(4-chlorphenyl)-äthyl]-piperazin vom Smp. 146-147° vor.

### Beispiel 7:

2.34 g 1-[2-(4-Chlorphenyl)-äthyl]-piperazin und 1.57 g Hünig-Base (= N-Aethyldiisopropylamin) werden in 40 ml Tetrahydrofuran vorgelegt. Dazu gibt man 2.36 g 4-Chloracetylaminobenzoesäurechlorid [s. J. Med. Chem. 28 (1985) 910] in kleinen Portionen zu. Die Lösung wird nach 30 Minuten bei 40° eingedampft. Der Rückstand wird an Kieselgel chromatographiert, und man kristallisiert aus Aceton um. Man erhält so das 1-(4-Chloracetylaminobenzoyl)-4-[2-(4-chlorphenyl)-äthyl]-piperazin vom Smp. 146-147°.

### Beispiel 8:

In analoger Weise wie im Beispiel 3 beschrieben wird aus 1.6 g 1-(4-Methyl-aminobenzoyl)-4-[2-(4-methylmercaptophenyl)-äthyl]-piperazin durch Umsetzung mit Chloracetylchlorid das 1-{4-[N-Chloracetyl-N-methyl-amino]-benzoyl}-4-[2-(4-methyl-mercaptophenyl)-äthyl]-piperazin vom Smp. 120-121° hergestellt.

Die Ausgangsverbindung wird wie folgt hergestellt: (a) 64.3 g 4-Chloräthyl-thiophenol werden mit 13.3 g Dimethylsulfat vorgelegt; unter Aussenkühlung (IT < 10⁰) werden dann 41 ml NaOH (25%) langsam zugetropft. Nach etwa 1 Std. wird die Aussenkühlung entfernt, vorsichtig aufgeheizt und 30 Min. bei 125⁰ gehalten. Die abgekühlte Lösung wird ausgeäthert und destilliert. Man erhält das 4-Methylmercaptophenyl-äthylchlorid; Sdp. 94-95⁰/0.1 mbar.

(b) Unter Stickstoff werden 59.6 g 4-Methylmercaptophenyl-äthylchlorid mit 75.7 g 1-Äthoxycarbonylpiperazin 1.5 Std. bei 130⁰ erwärmt. Man gibt noch 25.2 g 1-Äthoxycarbonylpiperazin hinzu, und nach 1.5 Std. werden noch 25.2 g 1-Äthoxycarbonylpiperazin hinzugefügt. Nach insgesamt 4.5 Std. wird die Lösung auf Eis-Wasser gegossen, mit conc. NaOH stark alkalisch gestellt und mit Äther extrahiert. Nach dem Eindampfen erhält man ein bewegliches Öl, welches chromatographisch auf Kieselgel gereinigt wird (Eluiermittel: Dichlormethan und Dichlormethan/Aceton 8:2). 90 g des so erhaltenen 1-[(4-Methylmercaptophenyl)-äthyl]-4-äthoxycarbonyl-piperazins werden mit 700 ml conc. HCl 17 Std. am Rückfluss gekocht. Die Lösung wird mit Eiswasser verdünnt, mit conc. NaOH alkalisch gestellt, mit Äthylacetat extrahiert, eingedampft und aus Äther/Petroläther umkristallisiert. Das 1-[2-(4-Methylmercaptophenyl)-äthyl]-piperazin schmilzt bei 52-54⁰.

(c) Das 1-(4-Methylaminobenzoyl)-4-[2-(4-methylmercaptophenyl)-äthyl]-piperazin wird aus 1.66 g 4-Methylamino-benzoesäure, 1.95 g N,N-Carbonyldiimidazol und 2.35 g 1-[2-(4-Methylmercaptophenyl)-äthyl]-piperazin in Dimethylformamid bei 85° hergestellt. Die Base wird via Hydrochlorid (Smp. 185°, Zers.) gereinigt und schmilzt bei 120-121°.

### Beispiel 9:

In analoger Weise wie im Beispiel 3 beschrieben wird aus 0.4 g 1-(4-Methyl-aminobenzoyl)-4-[2-(4-bromphenyl)-äthyl]-piperazin durch Umsetzung mit Chloracetylchlorid das 1-{4-[N-Chloracetyl-N-methyl-amino]-benzoyl}-4-[2-(4-bromphenyl)-äthyl]-piperazin vom Smp. 127-129° hergestellt.

Das Ausgangsmaterial wird so hergestellt: (a) Eine bei Raumtemperatur gerührte Lösung von 1.6 g p-N-Methylaminobenzoesäure in 25 ml wasserfreiem Dimethylformamid wird mit 1.9 g N,N-Carbonyldiimidazol versetzt. Nach etwa 15 Minuten hört die heftige Gasentwicklung auf. Man erwärmt auf 60°, und nach 5 Minuten werden 1.7 g 1-[2-(4-Bromphenyl)-äthyl]-piperazin zugegeben. Man rühst 45 Minuten bei 80-85° Ölbadtemperatur. Die abgekühlte Lösung wird mit 2 N Natronlauge und Wasser versetzt und gerührt. Es kristallisiert das 1-[4-(N-Methylamino)-benzoyl]-4-[2-(4-bromphenyl)-äthyl]-piperazin aus. Es wird abgenutscht und mit Wasser gewaschen; Smp. 93-94°.

### Beispiel 10:

In analoger Weise wie im Beispiel 3 beschrieben wird aus 1 g 1-(4-Äthyl- aminobenzoyl)-4-[2-(4-chlorphenyl)-äthyl]-piperazin (s. Europäische Patentanmeldung Nr. 489 690, Beispiel 23) durch Umsetzung mit Chloracetylchlorid das 1-{4-[N-Äthyl-N-chloracetyl-amino]-benzoyl}-4-[2-(4-chlorphenyl)-äthyl]-piperazin vom Smp. 118-120° hergestellt.

Das Ausgangsmaterial wird in analoger Weise wie im Beispiel 9a beschrieben aus 4-Ethylaminobenzoesäure und 1-[2-(4-Chlorphenyl)-äthyl]-piperazin hergestellt. Es schmilzt bei 99-101°.

### Beispiel 11:

In analoger Weise wie im Beispiel 6 beschrieben wird aus 1.35 g 1-[4-(2-Isopropyloxyäthyl)-aminobenzoyl]-4-[2-(4-chlorphenyl)-äthyl]-piperazin durch Umsetzung mit Chloracetylchlorid das ölige 1-{4-[N-(2-Isopropyloxyäthyl)-N-chloracetyl-amino]-benzoyl}-4-[2-(4-chlorphenyl)-äthyl]-piperazin hergestellt, IR /CH₂Cl₂): 1650 cm⁻¹ (breit); NMR (CDCl₃): 1.15 (t, 3H), 1.8 (m, 2H), 2.5 (m) und 2.6 (t) (zusammen 6H), 2.8 (t, 2H), 3.4 (m, 6H), 3.85 (m, 6H), 7.15 (d, 2H), 7.3 (m, 4H), 7.5 ppm (d, 2H).

### Beispiel 12:

2.5 g 4-Äthylmercaptoacetylamino-benzoesäure werden in 30 ml Dimethylformamid vorgelegt und mit 1.75 g N,N-Carbonyldiimidazol versetzt. Man rührt 10 Minuten bei Raumtemperatur. Bei 80° werden 1.75 g 1-[2-(4-Chlorphenyl)-äthyl]-piperazin dazugegeben. Man rührt 45 Minuten bei 80° weiter. Die Reaktionslösung wird eingedampft und das Öl chromatographiert. Das Hydrochlorid wird aus Äthanol-Äther umkristallisiert. Man erhält so das 1-[4-(Äthylmercaptoacetylamino)-benzoyl]-4-[2-(4-chlorphenyl)-äthyl]-piperazin-hydrochlorid vom Smp.229-231°.

Die Ausgangsverbindung wird wie folgt hergestellt: (a) 55.2 g Thioglykolsäure werden in einem Rundkolben vorgelegt und auf 140-150⁰ erwärmt. Dazu werden portionenweise 54.8 g 4-Aminobenzoesäure gegeben. 2 Std. bei 150⁰ halten, danach auf 100⁰ abkühlen lassen, mit 500 ml 2N NaOH versetzen und lösen. Durch Hyflo abfiltrieren und mit 80 ml conc. HCl ansäuern. Abnutschen, mit Wasser waschen, noch nass in 800 ml Methanol lösen und auf etwa 600 ml einengen. Es kristallisiert die 4-Mercaptoacetylamino-benzoesäure vom Smp. 220-222⁰ aus.

(b) 15 g 4-Mercaptoacetylamino-benzoesäure werden in 450 ml Tetrahydrofuran gelöst und dazu 39.2 g Pottasche und 17.24 ml Äthyljodid in 50 ml Tetrahydrofuran gegeben. Man rührt über Nacht und gibt 200 ml Wasser und etwas 2N NaOH hinzu. Man filiert die trübe Lösung, und das Filtrat wird mit conc. HCl angesäuert. Die abgenutschten gelben Kristalle werden aus Isopropanol umkristallisiert. Man erhält die 4-Äthylmercaptoacetylamino-benzoesäure vom Smp. 209-211⁰.

### Beispiel 13:

In analoger Weise wie in Beispiel 12 beschrieben und durch gewünschtenfalls anschliessende S-Oxidation, z.B. mit m-Chlorperbenzoesäure, kann kann man ferner herstellen:
(a) 1-[4-(Methylmercaptoacetylamino)-benzoyl]-4-[2-(4-chlorphenyl)-äthyl]-piperazin;
(b) 1-[4-(Methylsulfinylacetylamino)-benzoyl]-4-[2-(4-chlorphenyl)-äthyl]-piperazin und
(c) 1-[4-(Methylsulfonylacetylamino)-benzoyl]-4-[2-(4-chlorphenyl)-äthyl]-piperazin.

### Beispiel 14:

In analoger Weise wie in Beispiel 12 und 14a beschrieben kann man 1-{4-[N-Methyl-N-methylmercaptoacetyl-amino]-benzoyl}-4-[2-(4-chlorphenyl)-äthyl]-piperazin herstellen.

### Beispiel 14a:

1.3 g Äthylmercaptoessigsäure werden in 25 ml N,N-Dimethylformamid gelöst und mit 2.3 g N,N-Carbonyldiimidazol versetzt. Nach 15 Min. bei RT wird das Gemisch 5 Min bei 90⁰ AT erwärmt. Danach werden 3.5 g 1-[4-(N-Methylamino)**-**benzoyl]-4-[2-(4-chlorphenyl)äthyl]-piperazin (s. Europäische Patentanmeldung Nr. 489 690, Beispiel 9) dazugegeben, und man hält das Reaktionsgemisch 14 Std. lang bei 110⁰ AT. Eindampfen und das Öl auf Kieselgel chromatographieren (Eluiermittel: Dichlormethan/Aceton 8:2). Nach Behandlung mit alkoholischer Salzsäure erhält man das 1-{4-[N-Methyl-N-äthylmercaptoacetyl-amino]-benzoyl}-4-[2-(4-chlorphenyl)äthyl]-piperazin-hydrochlorid vom Smp. 163-164⁰.

### Beispiel 15:

In analoger Weise wie im Beispiel 12 wird aus 3.8 g 4-Acryloylamino-benzoesäure [=4-(Prop-2-en-1-on-1-yl-amino)-benzoesäure] und 4.4 g 1-[2-(4-Chlorphenyl)-äthyl]-piperazin 1-[4-(Acryloylamino)-benzoyl]-4-[2-(4-chlorphenyl)-äthyl]-piperazin vom Smp. 163-165° hergestellt.

### Beispiel 16:

2 g 1-(4-Aminobenzoyl)-4-[2-(4-chlorphenyl)-äthyl]-piperazin und 0.82 g Hünig-Base werden in 40 ml Tetrahydrofuran vorgelegt und dazu eine Lösung von 0.8 g 3-Chlorpropionsäurechlorid getropft. Die Lösung wird über Nacht stehen gelassen. Die entstandene Suspension wird eingedampft und in Methylenchlorid und Wasser aufgenommen. Aus der organischen Phase man erhält das 1-[4-(3-Chlorpropionylamino)-benzoyl]-4-[2-(4-chlorphenyl)-äthyl]-piperazin vom Smp. 190-191°.

### Beispiel 17:

In analoger Weise wie im Beispiel 16 wird aus 2 g 1-(4-Aminobenzoyl)-4-[2-(4-chlorphenyl)-äthyl]-piperazin und 0.9 g 4-Chlorbuttersäurechlorid 1-[4-(4-Chlorbutyrylamino)-benzoyl]-4-[2-(4-chlorphenyl)-äthyl]-piperazin vom Smp. 155-156° hergestellt.

### Beispiel 18:

In analoger Weise wie im Beispiel 16 wird aus 2 g 1-(4-Aminobenzoyl)-4-[2-(4-chlorphenyl)-äthyl]-piperazin und 0.98 g 5-Chlorvaleriansäurechlorid 1-[4-(5-Chlorvaleroylamino)-benzoyl]-4-[2-(4-chlorphenyl)-äthyl]-piperazin vom Smp. 115-116° hergestellt.

### Beispiel 19:

0.5 g 1-[4-(3-Chlorpropionylamino)-benzoyl]-4-[2-(4-chlorphenyl)-äthyl]-piperazin und 0.4 g Triäthylamin werden in 20 ml Methylenchlorid gelöst und 4 Stunden am Rückfluss gekocht, danach eingedampft und das 1-(4-Acryloylaminobenzoyl)-4-[2-(4-chlorphenyl)äthyl]-piperazin vom Smp. 163-165° gewonnen.

### Beispiel 20:

In analoger Weise wie in den Beispielen 1 bis 19 beschrieben kann man ferner herstellen:
(a) 1-[4-(N,N-Dimethylaminoacetylamino)-benzoyl]-4-[2-(4-chlorphenyl)-äthyl]-piperazin;
(b) 1-[4-(Piperidinoacetylamino)-benzoyl]-4-[2-(4-chlorphenyl)äthyl]-piperazin.

### Beispiel 21:

5g 1-{4-[N-(2-Isopropyloxyäthyl)-N-chloracetyl-amino]-benzoyl}-4-[2-(4-chlorphenyl)-äthyl]-piperazin (Beispiel 3) werden in 50 ml abs. Äthanol gelöst und mit 1.471 g Natriumthiophenolat versetzt. 1 Std. bei Raumtemperatur rühren lassen und nochmals 1 g Natriumthiophenolat zugeben. Weitere 3 Std. bei RT Rühren und danach die Reaktionslösung eindampfen. Der ölige Rückstand wird mit Äther extrahiert und die ätherischen Phasen nacheinander mit Wasser, Sodalösung und Wasser gewaschen, eingedampft und das hellgelbe Öl aus Äther und Hexan kristallisiert. Man erhält so das 1-{4-[N-(2-Isopropyloxyäthyl)-N-phenylmercaptoacetyl-amino]-benzoyl}-4-[2-(4-chlorphenyl)-äthyl]-piperazin vom Smp. 47-49⁰.

### Beispiel 22:

5 g 1-{4-[N-(2-Isopropyloxyäthyl)-amino]-benzoyl}-4-[2-(4-chlorphenyl)-äthyl]-piperazin (Beispiel 1) werden in 50 ml abs. Tetrahydrofuran gelöst und auf 3⁰ unter Rühren abgekühlt. Man gibt dazu 1.8 g Hünig-Base und anschliessend tropfenweise innerhalb von 5 Min. eine Lösung von 1.38 g Methoxyacetylchlorid in 5 ml abs. Tetrahydrofuran. 30 Min. bei RT Rühren lassen und Eindampfen. Der Rückstand wird in Wasser und Essigester aufgenommen, die organische Phase mit Natriumbicarbonat-Lösung und Wasser neutral gewaschen und eingedampft. Das braune Öl wird an Kieselgel chromatographiert. Man behandelt mit ätherischer Salzsäure. Nach dem Umkristallisieren aus Aceton und Äther erhält man das 1{4-[N-(2-Isopropyloxyäthyl)-N-methoxyacetyl-amino]-benzoyl}-4-[2-(4-chlorphenyl)-äthyl]-piperazin-hydrochlorid vom Smp. 149-151⁰.

### Beispiel 23:

3 g 1-{4-[N-(2-Isopropyloxyäthyl)-amino]-benzoyl}-4-[2-(4-chlorphenyl)-äthyl]-piperazin werden in 40 ml abs. Tetrahydrofuran gelöst und auf 3⁰ unter Rühren abgekühlt. Man gibt dazu 1.08 g Hünig-Base und anschliessend tropfenweise innerhalb von 5 Min. eine Lösung von 0.65 g Acrylsäurechlorid in 5 ml abs. Tetrahydrofuran. 30 Min. bei RT Rühren lassen und Eindampfen. Der Rückstand wird in Wasser und Essigester aufgenommen, die organische Phase mit Natriumbicarbonat-Lösung und Wasser neutral gewaschen und eingedampft. Das braune Öl wird an Kieselgel chromatographiert. Man behandelt mit ätherischer Salzsäure. Nach dem Umkristallisieren aus Aceton und Äther erhält man das 1{4-[N-(2-Isopropyloxyäthyl)-N-acryloyl-amino]-benzoyl}-4-[2-(4-chlorphenyl)-äthyl]-piperazin-hydrochlorid vom Smp. 132-134⁰.

### Beispiel 24:

In analoger Weise wie im Beispiel 23 beschrieben erhält man aus 3 g 1-{4-[N-(2-Isopropyloxyäthyl)-amino]-benzoyl}-4-[2-(4-chlorphenyl)-äthyl]-piperazin durch Umsetzung mit Acetylchlorid das 1-{4-[N-(2-Isopropyloxyäthyl)-N-acetyl-amino]-benzoyl}-4-[2-(4-chlorphenyl)-äthyl]-piperazin-hydrochlorid vom Smp. 142-144⁰.

### Beispiel 25:

In analoger Weise wie im Beispiel 23 beschrieben erhält man aus 3 g 1-{4-[N-(2-Isopropyloxyäthyl)-amino]-benzoyl}-4-[2-(4-chlorphenyl)-äthyl]-piperazin durch Umsetzung mit Oxalsäuremonomethylesterchlorid das 1-{4-[N-(2-Isopropyloxyäthyl)-N-methoxycarbonyl-carbonyl-amino]-benzoyl}-4-[2-(4-chlorphenyl)-äthyl]-piperazin-hydrochlorid vom Smp. 152-154⁰.

### Beispiel 25a:

In analoger Weise zu Beispiel 23 erhält man durch Umsetzung von 1-{4-[N-(2-Isopropyloxyäthyl)-amino]-benzoyl}-4-[2-(4-chlorphenyl)-äthyl]-piperazin mit Maleinsäuremethylesterchlorid das 1-{4-[N-(2-Isopropyloxyäthyl)-N-(3-methoxycarbonyl-prop-2-en(Z)-1-on-yl)-amino]-benzoyl}-4-[2-(4-chlorphenyl)-äthyl]-piperazin.

### Beispiel 25b:

In analoger Weise zu Beispiel 23 erhält man durch Umsetzung von 1-{4-[N-(2-Isopropyloxyäthyl)-amino]-benzoyl}-4-[2-(4-chlorphenyl)-äthyl]-piperazin mit Fumarsäuremethylesterchlorid das 1-{4-[N-(2-Isopropyloxyäthyl)-N-(3-methoxycarbonyl-prop-2-en(E)-1-on-yl)-amino]-benzoyl}-4-[2-(4-chlorphenyl)-äthyl]-piperazin.

### Beispiel 25c:

In analoger Weise wie im Beispiel 23 beschrieben erhält man aus 1-{4-[N-(2-Isopropyloxyäthyl)-amino]-benzoyl}-4-[2-(4-chlorphenyl)-äthyl]-piperazin und Maleinsäureäthylesterchlorid das 1-{4-[N-(2-Isopropyloxyäthyl)-N-(3-äthoxycarbonyl-prop-2-en(Z)-1-on-yl)-amino]-benzoyl}-4-[2-(4-chlorphenyl)-äthyl]-piperazin. Das Hydrochlorid davon schmilzt bei 181-183°.

### Beispiel 25d:

In analoger Weise wie im Beispiel 23 beschrieben erhält man aus 1{4-[N-(2-Isopropyloxyäthyl)-amino]-benzoyl}-4-[2-(4-chlorphenyl)-äthyl]-piperazin und Fumarsäureäthylesterchlorid das 1-{4-[N-(2-Isopropyloxyäthyl)-N-(3-äthoxycarbonyl-prop-2-en(E)-1-on-yl)-amino]-benzoyl}-4-[2-(4-chlorphenyl)-äthyl]-piperazin-hydrochlorid vom Smp. 180-182⁰.

### Beispiel 26:

2.18 g 4-Chlorbenzylpiperazin werden in 40 ml abs. Tetrahydrofuran gelöst. Unter Rühren werden nacheinander 1.73 g Hünig-Base und 2.64 g 4-Chloracetylamino-benzoesäurechlorid dazugegeben. Die interne Temperatur steigt auf ca. 38⁰ an. Nach 30 min wird die Lösung eingedampft. Der ölige Rückstand wird mit Wasser digeriert. Man erhält so das 1-[4-(N-Chloracetyl-amino)-benzoyl]-4-(4-chlorbenzyl)-piperazin vom Smp. 205-207⁰.

### Beispiel 27:

In analoger Weise wie im Beispiel 26 beschrieben erhält man aus 2.03 g 1-Cinnamylpiperazin [= 1-(3-Phenylprop-2-enyl)-piperazin] und 2.21 g 4-Chloracetyl-amino-benzoesäurechlorid das 1-[4-(N-Chloracetyl-amino)-benzoyl]-4-[3-phenylprop-2-enyl]-piperazin vom Smp. 141-144⁰.

### Beispiel 28:

In analoger Weise wie im Beispiel 26 beschrieben erhält man aus 1.54 g 1-[3-(4-Chlorphenylpropyl]-piperazin und 1.24 g 4-Chloracetyl-amino-benzoesäurechlorid das 1-[4-(N-Chloracetyl-amino)-benzoyl]-4-[3-(4-chlorphenyl)-propyl]-piperazin vom Smp. 158-161⁰.

### Beispiel 29:

In analoger Weise wie im Beispiel 22 beschrieben erhält man aus 2 g 1-(2-Amino-benzoyl)-4-[2-(4-chlorphenyl)-äthyl]-piperazin durch Umsetzung mit Chloracetylchlorid das 1-[2-(N-Chloracetyl-amino)-benzoyl]-4-[2-(4-chlorphenyl)-äthyl]-piperazin vom Smp. 108-109⁰.

Das Ausgangsmaterial wird wie folgt hergestellt: 10 g Isatosäureanhydrid werden in 100 ml Dioxan suspendiert. Diese Suspension wird auf 60⁰ IT aufgewärmt, und innerhalb von 10 Min. werden 13.74 g 1-[2-(4-chlorphenyl)-äthyl]-piperazin in 20 ml Dioxan zugetropft. 30 Min. am Rückfluss kochen und danach Eindampfen. Den Rückstand in Äther lösen und mit Sodalösung (1%) waschen. Äther-Lösung eindampfen und das gelbe Öl aus Hexan umkristallisieren. Es liegt das 1-(2-Amino-benzoyl)-4-[2-(4-chlorphenyl)-äthyl]-piperazin vom Smp. 78-80⁰ vor.

### Beispiel 30:

In analoger Weise wie im Beispiel 26 beschrieben erhält man aus 2 g 1-[4-(4-Chlorphenyl)-butyl]-piperazin (s. US Patent 4 725 597) und 1.93 g 4-Chloracetyl-amino-benzoesäurechlorid das 1-[4-(N-Chloracetyl-amino)-benzoyl]-4-[4-(4-chlorphenyl)butyl]-piperazin vom Smp. 158-160⁰.

### Beispiel 31:

In analoger Weise wie im Beispiel 26 beschrieben erhält man aus 2.5 g 1-[2-(4-Chlorphenyl)-äthyl]-piperazin und 2.72 g 3-Chloracetyl-amino-benzoesäurechlorid (s. US Patent 4 093 739) das 1-[3-(N-Chloracetyl-amino)-benzoyl]-4-[2-(4-chlorphenyl)äthyl]-piperazin vom Smp. 125-126⁰.

### Beispiel 32:

In analoger Weise wie im Beispiel 26 beschrieben erhält man aus 2.33 g 1-(2-Chlorphenyl)-piperazin und 2.44 g 4-Chloracetyl-amino-benzoesäurechlorid das 1-[4-(N-Chloracetyl-amino)-benzoyl]-4-(2-chlorphenyl)-piperazin vom Smp. 177-179⁰.

### Beispiel 33:

0.42 g Natriumhydrid-Dispersion 50% werden in 25 ml abs. Dimethylformamid vorgelegt. Bei RT wird dazu eine Lösung von 1-[4-(N-Dimethylcarbamoyl-amino)-benzoyl]-4-[2-(4-chlorphenyl)-ethyl]-piperazin in 30 ml abs. Dimethylformamid getropft. Nach etwa 15 min setzt eine Gasentwicklung ein. Nach 5 min bei 60⁰ IT ist sie beendet. Bei 30⁰ wird eine Lösung von 2-Isopropyloxyäthylbromid in 5 ml abs. Dimethylformamid hinzugegeben. Man rührt 1h bei RT und 4h bei 60⁰ weiter. Es wird eingedampft und an Kieselgel mit alkoholischer Salzsäure chromatographiert; so erhält man das 1-{4-[N-(2-Isopropyloxyäthyl)-N-(N′,N′-dimethylcarbamoyl)-amino]-benzoyl}-4-[2-(4-chlorphenyl)-äthyl]-piperazin-hydrochlorid vom Smp. 179-181⁰.

### Beispiel 34:

In analoger Weise wie im Beispiel 26 beschrieben erhält man aus 2 g 1-(4-Chlorphenyl)-piperazin und 2.36 g 4-Chloracetyl-amino-benzoesäurechlorid das 1-[4-(N-Chloracetyl-amino)-benzoyl]-4-(4-chlorphenyl)-piperazin vom Smp. 218-221⁰.

### Beispiel 35:

In analoger Weise wie im Beispiel 26 beschrieben erhält man aus 5.02 g 1-(3-Chlorphenyl)-piperazin und 6.51 g 4-Chloracetyl-amino-benzoesäurechlorid das 1-[4-(N-Chloracetyl-amino)-benzoyl]-4-[(3-chlorphenyl)-piperazin vom Smp. 174-175⁰.

### Beispiel 36:

In analoger Weise wie im Beispiel 26 beschrieben erhält man aus 1.66 g 1-[(4-Chlorphenyl)-äthyl]-piperazin und 2 g 4-Chloracetyl-amino-phenylessigsäurechlorid [s. J. Amer. Chem. Soc. 41 (1919) 469] das 1-[4-(N-Chloracetyl-amino)-phenylacetyl]-4-[2-(4-chlorphenyl)-äthyl]-piperazin vom Smp. 163-165⁰.

### Beispiel 37:

3.4 g 1-(4-Amino-benzoyl)-4-[2-(4-chlorphenyl)-piperazin werden in 100 ml abs. Dichlormethan gelöst. Dazu gibt man 1.2 g Triäthylamin und tropfenweise 1.3 g Dimethylcarbamoylchlorid in 5 ml abs. Dichlormethan. Nach 20 Std. RT werden 0.12 g 4-Dimethylaminopyridin zugegeben, und es wird weitere 4 Std. bei RT gerührt. Die organische Phase wird mit Wasser gewaschen und eingedampft. Das hochvisköse Öl kann als Hydrochlorid kristallisiert werden. Man erhält so das 1-[4-(N-Dimethylcarbamoyl-amino)-benzoyl]-4-[2-(4-chlorphenyl)-ethyl]-piperazin-hydrochlorid vom Smp. 245-247⁰.

### Beispiel 38:

In analoger Weise wie im Beispiel 26 beschrieben erhält man aus 1.45 g 1-(4-Chlorphenyl)-piperazin und 1.99 g 4-Chloracetyl-amino-phenylessigsäurechlorid das 1-[4-(N-Chloracetyl-amino)-phenylacetyl]-4-(4-chlorphenyl)-piperazin vom Smp. 195-197⁰.

### Beispiel 39:

1.55 g 1-{4-[N-(2-Isopropyloxyäthyl)-N-phenylmercaptoacetyl-amino]-benzoyl}-4-[2-(4-chlorphenyl)-äthyl]-piperazin (Beispiel 21) werden in 50 ml Dichlormethan gelöst und gerührt. Dazu gibt man 0.56 g m-Chlorperbenzoesäure (90%) wobei die IT auf 26⁰ steigt. Nach 30 Min. werden noch 0.1g m-Chlorperbenzoesäure (90%) dazugegeben. Nach weiteren 15 Min. wird die Lösung eingedampft. Der Rückstand wird in Wasser und Essigester aufgenommen, die organische Phase mit Natriumbicarbonat-Lösung und Wasser neutral gewaschen und eingedampft. Der Rückstand wird mit Äther digeriert. Man erhält so das 1-{4-[N-(2-Isopropyloxyäthyl)-N-phenylsulfonyl-acetyl-amino]benzoyl}-4-[2-(4-chlorphenyl)-äthyl]-piperazin vom Smp. 115-116⁰.

### Beispiel 40:

In analoger Weise wie im Beispiel 23 beschrieben erhält man aus 1 g 1-{4-[N-(2-Isopropyloxyäthyl)-amino]-benzoyl}-4-[2-(4-chlorphenyl)-äthyl]-piperazin (Beispiel 1) durch Umsetzung mit Malonsäuremonomethylesterchlorid das 1-{4-[N-(2-Isopropyloxyäthyl)-N-methoxycarbonylacetyl-amino]-benzoyl}-4-[2-(4-chlorphenyl)-äthyl]-piperazin-hydrochlorid vom Smp. 77-79⁰.

### Beispiel 41:

In analoger Weise wie im Beispiel 23 beschrieben erhält man aus 5 g 1-{4-[N-(2-Isopropyloxyäthyl)-amino]-benzoyl}-4-[2-(4-chlorphenyl)-äthyl]-piperazin (Beispiel 21) durch Umsetzung mit Acetoxyessigsäurechlorid das 1-{4-[N-(2-Isopropyloxyäthyl)-N-acetoxyacetyl-amino]-benzoyl}-4-[2-(4-chlorphenyl)-äthyl]-piperazin-hydrochlorid vom Smp. 130-132⁰.

### Beispiel 42:

1.2 g 1-{4-[N-(2-Isopropyloxyäthyl)-N-acetoxyacetyl-amino]-benzoyl}-4-[2-(4-chlorphenyl)-äthyl]-piperazin (Beispiel 41) werden in 20 ml Äthanol gelöst. Dazu gibt man 1.5 ml 2N Natronlauge in 5 ml Wasser und rührt bei RT 1 h. Nach dem Eindampfen wird der Rückstand in Essigester aufgelöst und mit Wasser gewaschen. Nach Eindampfen und Behandlung mit ätherischer Salzsäure erhält man das 1-{4-[N-(2-Isopropyloxyäthyl)-N-hydroxyacetyl-amino]-benzoyl}-4-[2-(4-chlorphenyl)-äthyl]-piperazin-hydrochlorid vom Smp. 99-101⁰.

### Beispiel 43:

1.2 g 1-{4-[N-(2-Isopropyloxyäthyl)-amino]-benzoyl}-4-[2-(4-chlorphenyl)-äthyl]-piperazin werden in 15 ml Toluol gelöst und mit 1.1 g Pottasche versetzt. Man rührt 10 Min. bei RT und tropft danach 1.9 g Chlorameisensäurebenzylester in 4 ml Toluol dazu. Über Nacht bei RT rühren lassen, eindampfen, den Rückstand zwischen Dichlormethan und Wasser verteilen, eindampfen und auf Kieselgel chromatographieren (Eluiermittel: Dichlormethan/Aceton 9:1). Nach Behandlung mit alkoholischer Salzsäure erhält man das 1-{4-[N-(2-Isopropyloxyäthyl)-benzyloxycarbonyl-amino]-benzoyl}-4-[2-(4-chlorphenyl)-äthyl]-piperazin-hydrochlorid vom Smp. 135-137⁰.

### Beispiel 44:

0.85 g 1-{4-[N-(3-Äthoxypropyl)-amino]-benzoyl}-4-[2-(4-chlorphenyl)-äthyl]-piperazin werden in 20 ml abs. Tetrahydrofuran gelöst und nacheinander mit 0.33 g Hünig-Base und 1.34 ml Chlorameisensäurebenzylester in Toluol versetzt. Nach 30 Min. Rühren bei RT eindampfen, mit Essigester ausschütteln, mit Salzsäure versetzen und wieder eindampfen. Nach Umkristallisieren aus Hexan/Äther erhält man das 1-{4-[N-(3-Äthoxypropyl)-benzyloxycarbonyl-amino]-benzoyl}-4-[2-(4-chlorphenyl)-äthyl]-piperazin-hydrochlorid vom Smp. 113-115⁰.

### Beispiel 45:

200 g 1-[N-(tert.-Butyloxycarbonyl-amino)-benzoyl]-4-[2-(4-chlorphenyl)-äthyl]-piperazin werden in 700 ml N,N-Dimethylformamid gelöst und mit 34 g pulv. KOH versetzt. Man rührt 30 Min. bei RT; danach wird innerhalb von 15 Min. eine Lösung von 136 g O-(2-Isopropyloxyäthyl)-tosylat in 150 ml N,N-Dimethylformamid dazugetropft. 30 Min. bei RT und 4 Std. bei 45-50⁰ rühren, eindampfen, ausäthern, mit Wasser waschen und eindampfen. Nach Behandlung mit alkoholischer Salzsäure erhält man das 1-{4-[N-(2-Isopropoxyäthyl)-t-butyloxycarbonyl-amino]-benzoyl}-4-[2-(4-chlorphenyl)-äthyl]-piperazin-hydrochlorid vom Smp. 182-184⁰.

### Beispiel 46:

2 g 4-(Äthylsulfinylacetyl-amino)-benzoesäure werden mit 1.5 g N,N-Carbonyldiimidazol in 30 ml abs. Dimethylformamid suspendiert und 20 Min. bei 80⁰ AT erwärmt. Man erhält eine klare Lösung, welcher bei 80⁰ 1.75 g 1-[2-(4-Chlorphenyl)-äthyl]-piperazin zugegeben wird. Nach 30 min bei 80⁰ wird die Lösung eingedampft. Der Rückstand wird in Dichlormethan gelöst und mit Wasser gewaschen, an Kieselgel chromatographiert und aus Äthanol kristallisiert. Man erhält so 1-[4-(N-Aethylsulfinylacetyl-amino)-benzoyl]-4-[2-(4-chlorphenyl)-äthyl]-piperazin vom Smp. 163-164⁰.

Die Ausgangsverbindung wird wie folgt hergestellt: 9.2 g 4-(Äthylmercaptoacetyl-amino)-benzoesäure werden in 200 ml abs. Tetrahydrofuran gelöst. Bei 35⁰ wird eine Lösung von 3-Chlorperbenzoesäure in 40 ml abs. Tetrahydrofuran zugetropft. Über Nacht bei RT rühren lassen. Die Suspension wird abgenutscht und das kristalline Produkt wird aus Äthanol umkristallisiert. Die 4-(Äthylsulfinylacetyl-amino)-benzoesäure schmilzt bei 203-205⁰.

### Beispiel 47:

1.4 g 1-[4-(N-Aethylsulfinylacetyl-amino)-benzoyl]-4-[2-(4-chlorphenyl)-äthyl]-piperazin (Beispiel 46) werden in 50 ml Dimethylformamid gelöst und bei RT mit einer Lösung von 3-Chlorperbenzoesäure (85%) in 30 ml Tetrahydrofuran tropfenweise versetzt. Die klare Lösung wird über Nacht bei RT gerührt. Eindampfen, den Rückstand in Wasser lösen und neutral stellen, mit Dichlormethan extrahieren, an Kieselgel chromatographieren und aus Äther-Petroläther-Alkohol kristallisieren. Man erhält so das 1-[4-(N-Aethylsulfonylacetyl-amino)-benzoyl]-4-[2-(4-chlorphenyl)-äthyl]-piperazin vom Smp. 135-136⁰.

### Beispiel 48:

1.15 g N-(2-Isopropyloxyäthyl)-isatosäureanhydrid (= N-(2-Isopropyloxyäthyl)-dihydro-2,4-dioxo-3,1-benzoxazin) werden in 20 ml Dioxan gelöst, und man gibt 0.942 g 1-[2-(4-Chlorphenyl)-äthyl]-piperazin hinzu. Man rührt bei 50⁰ IT, wobei sofort CO₂ entweicht. Nach 1h wird die Reaktionslösung eingedampft, der Rückstand in Essigester aufgenommen und die organische Phase mit Wasser neutral gewaschen. Eindampfen und an Kieselgel chromatographieren. Nach Behandlung mit ätherischer Salzsäure erhält man das 1-{2-[N-(2-Isopropyloxyäthyl)-amino]-benzoyl}-4-[2-(4-chlorphenyl)-äthyl]-piperazin-hydrochlorid vom Smp. 237-240⁰.

Die Ausgangsverbindung wird wie folgt hergestellt: 3 g Isatosäureanhydrid werden in 50 ml dest. Hexametapol (= Hexamethyl-phosphorsäuretriamid) gelöst. Die Lösung wird auf 5⁰ gekühlt und mit 0.96 g NaH 55% (mit Hexan entölt) versetzt. Man lässt 30 Min. bei RT rühren und gibt danach 5.67 g O-(2-Isopropyloxyäthyl)-tosylat hinzu. 30 Min. bei RT und 1 Std. bei 50⁰ rühren lassen. Über Nacht stehen lassen, auf Eis giessen, die organische Schicht abtrennen und mit Wasser waschen. Man dampft ein, digeriert den Rückstand mit Äther und saugt ab. So erhält man das N-(2-Isopropyloxyäthyl)-isatosäureanhydrid, Smp. 107-108⁰.

### Beispiel 49:

0.82 g 1-{2-[N-(2-Isopropyloxyäthyl)-amino]-benzoyl}-4-[2-(4-chlorphenyl)-äthyl]-piperazin (Beispiel 48) werden in 30 ml abs. Tetrahydrofuran vorgelegt. Dazu gibt man unter Aussenkühlung 0.295 g Hünig-Base und anschliessend eine Lösung von 0.236 g Chloracetylchlorid in 2 ml abs. Tetrahydrofuran. Nach 30 Min. Rühren bei RT ist die Reaktion beendet. Eindampfen und den Rückstand mit Essigester extrahieren, mit Wasser waschen und wieder eindampfen. Nach Behandlung mit ätherischer Salzsäure erhält man das 1-{2-[N-(2-Isopropyloxyäthyl)-N-chloracetyl-amino]-benzoyl}-4-[2-(4-chlorphenyl)-äthyl]-piperazin-hydrochlorid vom Smp. 112-113⁰.

### Beispiel 50:

In analoger Weise wie im Beispiel 26 beschrieben erhält man aus 7.72 g 1-[2-(4-Chlorphenyl)-äthyl]-piperazin und 8 g 4-(N-Chloracetyl-N-methyl-amino)-benzoesäurechlorid das 1-[4-(N-Chloracetyl-N-methyl-amino)-benzoyl]-4-[2-(4-chlorphenyl)-äthyl]-piperazin, das nach chromatographischer Reinigung an Kieselgel (Eluiermittel: CH₂Cl₂/Aceton) einen Smp. von 113-115⁰ aufweist.

Die Ausgangsverbindung wird wie folgt hergestellt: 30 g 4-(N-Methylamino)-benzoesäure werden in 500 ml Dichlormethan mit 30.7 g Hünig-Base und bei 5⁰ mit 24.6 g Chloracetylchlorid in 50 ml Dichlormethan versetzt. Nach 2 Std. Rühren bei RT wird die Supsension eingedampft, der Rückstand mit Wasser digeriert, abgenutscht und mit 1N Salzsäure gewaschen. Mit Äther digerieren und abnutschen. Man erhält die 4-(N-Chloracetyl-N-methyl-amino)-benzoesäure vom Smp. (186°) 195-197⁰. 10 g davon werden in 150 ml Methylenchlorid mit 5 Tropfen Pyridin mit 15 ml Thionylchlorid 2 Std. am Rückfluss gekocht, eingedampft und aus Hexan umkristallisiert. Das erhaltene 4-(N-Chloracetyl-N-methyl-amino)-benzoesäurechlorid schmilzt bei 61-63⁰.

### Beispiel 51:

0.9 g Glycinäthylester-hydrochlorid werden in 20 ml Äthanol gelöst und mit 0.87 g Hünig-Base und 0.5 g 1-{4-[N-(2-Isopropyloxyäthyl)-N-chloracetyl-amino]-benzoyl}-4-[2-(4-chlorphenyl)-äthyl]-piperazin (Beispiel 3) versetzt. Man kocht 24h am Rückfluss. Eindampfen, den Rückstand in Essigester lösen und mit Wasser neutral waschen. Nach dem Eindampfen und Behandlung mit ätherischer Salzsäure erhält man das 1-{4-[N-(2-Isopropyloxyäthyl)-N-(N′-äthoxycarbonylmethylamino)-acetyl-amino]-benzoyl}-4-[2-(4-chlorphenyl)-äthyl]-piperazin-hydrochlorid vom Smp. 124-126⁰.

### Beispiel 52:

Man legt 65 ml Trifluoressigsäure in 100 ml Dichlormethan vor. Dazu wird innerhalb von 5 min eine Lösung von 7.2 g 1-{3-[N-(2-Isopropyloxyäthyl)-N-t-butoxy-carbonyl-amino]-benzoyl}-4-[2-(4-chlorphenyl)-äthyl]-piperazin gegeben. Nach 2h bei RT wird die Reaktionslösung eingedampft, der Rückstand mit Natronlauge neutralisiert und ausgeäthert. Nach Eindampfen und Behandlung mit ätherischer Salzsäure erhält man das 1-[3-(2-Isopropyloxyäthyl-amino)-benzoyl]-4-[2-(4-chlorphenyl)-äthyl]-piperazin-hydrochlorid vom Smp. 157-159⁰.

Ausgangsverbindungen: (a) 3-Acetylaminobenzoesäure wird mit Thionylchlorid ins Säurechlorid überführt [Smp. 101-102⁰], und letzteres dann mit 1 -[2-(4-Chlorphenyl)-äthyl]-piperazin kondensiert, wobei man das 1 -(3-Acetylamino-benzoyl)-4-[2-(4-chlorphenyl)-äthyl]-piperazin als Schaum erhält. Dieses wird mit (BOC)₂O zum 1-{3-[(N-Acetyl-N-t-butyloxycarbonyl)-amino]-benzoyl}-4-[2-(4-chlorphenyl)äthyl]-piperazin vom Smp. 113-114⁰ umgesetzt. Die nachfolgende Spaltung mit Dimethylamino-äthylamin führt zu einem Schaum, der dem 1-[3-(N-t-Butyloxycarbonyl-amino)-benzoyl]-4-[2-(4-chlorphenyl)-äthyl]-piperazin entspricht.

(b) 6 g 1-[3-(N-t-Butyloxycarbonyl-amino)-benzoyl]-4-[2-(4-chlorphenyl)-äthyl]-piperazin und 4. 18 g 2-Isopropyloxyäthyl-p-toluolsulfonat werden in DMSO in Anwesenheit von 1.01 g fein pulv. KOH umgesetzt. Das so erhaltene ölige 1-{3-[N-(2-Isopropyloxyäthyl)-N-t-butoxycarbonyl-amino]-benzoyl}-4-[2-(4-chlorphenyl)äthyl] -piperazin wird direkt weiter umgesetzt.

### Beispiel 53:

2.77 g 1-[3-(2-Isopropyloxyäthyl-amino)-benzoyl]-4-[2-(4-chlorphenyl)-äthyl]-piperazin werden in 50 ml abs. Tetrahydrofuran gelöst und bei RT mit 1.07 g Hünig-Base und 0.794 g Chloracetylchlorid in 5 ml abs. Tetrahydrofuran gerührt. Nach etwa 15 min wird die dünne Suspension eingedampft, in Essigester gelöst und mit Wasser neutral gewaschen. Nach Eindampfen, Chromatographie des Rückstands an Kieselgel (Eluiermittel: Essigester) und Behandlung mit ätherischer Salzsäure erhält man das 1-{3-[N-(2-Isopropyloxyäthyl)-N-chloracetyl-amino]-benzoyl}-4-[2-(4-chlorphenyl)-äthyl]-piperazin-hydrochlorid vom Smp. 115-117⁰.

### Beispiel 54a:

Analog Beispiel 22 wird das 1-[4-(2-Dimethylaminoäthyl-amino)-benzoyl]-4-[2-(4-chlorphenyl)-äthyl]-piperazin (Beispiel 54b) mit Chloracetylchlorid umgesetzt, wobei man das 1-{4-[N-(2-Dimethylaminoäthyl)-N-chloracetyl-amino]-benzoyl}-4-[2-(4-chlorphenyl)-äthyl]-piperazin erhält. Das Hydrochlorid davon schmilzt bei 215-216° (Zers.).

### Beispiel 54b:

1.9 g 1-{4-[N-(2-Dimethylaminoäthyl)-N-benzyloxycarbonyl-amino]-benzoyl}-4-[2-(4-chlorphenyl)-äthyl]-piperazin werden in 30 ml Methanol in Anwesenheit von 0.4 g Palladium-Kohle hydriert. Abnutschen, eindampfen, mit 2N NaOH alkalisch stellen und ausäthern. Eindampfen und aus Äther umkristallisieren. Das 1-[4-(2-Dimethylaminoäthyl-amino)-benzoyl]-4-[2-(4-chlorphenyl)-äthyl]-piperazin schmilzt bei 74-78⁰. Mit alkoholischer Salzsäure erhält man das 1-[4-(2-Dimethylamïnoäthyl-amino)-benzoyl]-4-[2-(4-chlorphenyl)-äthyl]-piperazin-hydrochlorid vom Smp. >275⁰.

Die Ausgangsverbindung wird wie folgt hergestellt: 0.5 g NaH-Dispersion 50% werden in 15 ml abs. N,N,-Dimethylformamid suspendiert. Dazu tropft man eine Lösung von 4.7 g 1-[4-(N-Benzyloxycarbonyl-amino)-benzoyl}-4-[2-(4-chlorphenyl)-äthyl]-piperazin (s. Europäische Patentanmeldung Nr. 489 690, Beispiel 28) in 30 ml N,N-Dimethylformamid. Nach 30 min gibt man 1.18 g 1-Chlor-2-dimethylaminoäthan in 1 ml N,N,-Dimethylformamid hinzu. Über Nacht bei RT rühren lassen. Eindampfen, mit Wasser versetzen und mit Essigester ausschütteln, eindampfen und auf Kieselgel chromatographieren (Eluiermittel: Dichlormethan/Aceton/Methanol 70:30:8). Das viskose Öl entspricht dem 1-{4-[N-(2-Dimethylaminoäthyl)-N-benzyloxycarbonyl-amino]-benzoyl}-4-[2-(4-chlorphenyl)-äthyl]-piperazin und wird direkt weiter umgesetzt.

### Beispiel 55:

0.8 g 1-(2-Phenyl-äthyl)-piperazin werden in 15 ml Dichlormethan gelöst und nacheinander mit 0.56 g Hünig-Base und 1.5 g 4-(N-2-Isopropyloxyäthyl-N-chloracetylamino)-benzoylchlorid in 5 ml Dichlormethan versetzt. Über Nacht rühren lassen, eindampfen, zwischen Dichlormethan und Wasser verteilen, die organische Phase eindampfen und auf Kieselgel chromatographieren (Eluiermittel: Dichlormethan/Aceton 50:8 und 50:10). Nach Behandlung mit alkoholischer Salzsäure erhält man das 1-{4-[N-(2-Isopropyloxyäthyl)-N-chloracetyl-amino]-benzoyl}-4-(2-phenyl-äthyl)piperazin-hydrochlorid vom Smp. 170-171⁰.

Die Ausgangsverbindung wird wie folgt hergestellt: (a) 22.4 g 4-Aminobenzoesäureäthylester werden in 220 ml Pyridin und 220 ml Acetanhydrid über Nacht bei RT stehen gelassen. Eindampfen, den Rückstand in Wasser und Dichlormethan aufnehmen, die organische Phase neutral waschen, einengen und mit Äther verdünnen. Der auskristallisierte 4-Acetylamino-benzoesäureäthylester wird abgenutscht; Smp. 110-111⁰.

(b) 8.3 g davon werden in 100 ml Acetonitril gelöst und mit 0.5 g 4-Dimethylaminopyridin versetzt. Dazu tropft man innerhalb von 5 Min. 10.5 g (BOC)₂O in 50 ml Acetonitril. Über Nacht bei RT stehen lassen, danach weitere 1.5 g (BOC)₂O hinzugeben und noch 6 Std. stehen lassen. Es liegt das 4-(N-Acetyl-N-t-butyloxycarbonyl-amino)-benzoesäureäthylester in Lösung vor. Man gibt zu dieser Lösung 8 g 2-Diäthylaminoäthylamin und lässt das Gemisch über Nacht stehen. Eindampfen, den Rückstand in Essigester aufnehmen und mit Wasser waschen, einengen, abnutschen und mit Petroläther/Essigester waschen. So erhält man den 4-t-Butyloxycarbonyl-amino-benzoesäureäthylester, Smp. 149-150⁰.

(c) Man löst 2.6 g davon in 15 ml N,N-Dimethylformamid und gibt 0.7 g pulv. KOH dazu. 15 Min. bei RT rühsen lassen, danach 3 g O-(2-Isopropyloxyäthyl)-tosylat in 5 ml N,N-Dimethylformamid rasch zutropfen, anschliessend 5.5 Std. bei 45-50⁰ IT und über Nacht bei RT halten. Auf Eis giessen, mit 2N HCl ansäuern und mit Äther extrahieren, eindampfen und auf Kieselgel chromatographieren (Eluiermittel: Dichlormethan). So erhält man das 4-[N-t-Butyloxycarbonyl-N-(2-Isopropyloxyäthyl)-amino]-benzoesäureäthylester als ein Öl. Es wird direkt weiter verwendet.

(d) 7.7 g davon werden in 100 ml Dichlormethan gelöst, mit 60 ml Trifluoressigsäure versetzt und 20 Std. bei RT gerührt. Eindampfen, den Rückstand mit Wasser versetzen, mit Ammoniak alkalisch stellen und mit Dichlormethan extrahieren. Man dampft ein und verseift den rohen 4-[N-(2-Isopropyloxyäthyl)] -benzoesäureäthylester in 50 ml Äthanol mit 21,6 ml 2N NaOH über Nacht bei RT und dann noch 4 Std. bei 50-55⁰. Man gibt 21.6 ml 2N HCl zu. Der Niederschlag wird abgenutscht. Man erhält die 4-[N-(2-Isopropyloxyäthyl)-amino]-benzoesäure vom Smp. 116-118⁰.

(e) 0.22 g davon in 10 ml Dichlormethan werden mit einer Lösung von 0.17 g Chloracetylchlorid in 3 ml Dichlormethan versetzt. Ueber Nacht bei RT stehen lassen, eindampfen, den Rückstand in Äther aufnehmen, mit Wasser waschen, auf etwa 15 ml einengen und mit Petroläther versetzen. Das Kristallisat wird abgenuscht. Man erhält so die 4-[N-(2-Isopropyloxyäthyl)-N-chloracetyl-amino]-benzoesäure vom Smp. 129-130⁰.

### Beispiel 56:

Analog Beispiel 55 werden 1.5 g 4-[N-(2-Isopropyloxyäthyl)-N-chloracetyl-amino]-benzoylchlorid mit 1.07 g [2-(4-Bromphenyl)-äthyl]-piperazin umgesetzt, wobei man das 1-{4-[N-(2-Isopropyloxyäthyl)-N-chloracetyl-amino]-benzoyl}-4-[2-(4-bromphenyl)-äthyl]-piperazin vom Smp. 92-93⁰ erhält.

### Beispiel 57:

Analog Beispiel 55 werden 1.5 g 4-[N-(2-Isopropyloxyäthyl)-N-chloracetyl-amino]-benzoylchlorid mit 0.9 g [2-(4-Fluorphenyl)-äthyl]-piperazin umgesetzt, wobei man das 1-{4-[N-(2-Isopropyloxyäthyl)-N-chloracetyl-amino]-benzoyl}-4-[2-(4-fluorphenyl)-äthyl]-piperazin erhält. Das Hydrochlorid davon schmilzt bei 166-167⁰.

### Beispiel 58:

Analog Beispiel 55 werden 1.5 g 4-[N-(2-Isopropyloxyäthyl)-N-chloracetyl-amino]-benzoylchlorid mit 0.95 g [2-(4-Methylmercaptophenyl)-äthyl]-piperazin umgesetzt, wobei man das 1-{4-[N-(2-Isopropyloxyäthyl)-N-chloracetyl-amino]-benzoyl}-4-[2-(4-methylmercaptophenyl)-äthyl]-piperazin erhält. Das Hydrochlorid davon schmilzt bei 115-116⁰.

### Beispiel 59:

Analog Beispiel 55 werden 1.5 g 4-[N-(2-Isopropyloxyäthyl)-N-chloracetyl-amino]-benzoylchlorid mit 0.88 g [2-(4-Methoxyphenyl)-äthyl]-piperazin umgesetzt, wobei man das 1-{4-[N-(2-Isopropyloxyäthyl)-N-chloracetyl-amino]-benzoyl}-4-[2-(4-methoxyphenyl)-äthyl]-piperazin erhält. Das Hydrochlorid davon schmilzt bei 139-140°.

### Beispiel 59a:

Analog Beispiel 55 werden 1.5 g 4-[N-(2-Isopropyloxyäthyl)-N-chloracetyl-amino]-benzoylchlorid mit 0.8 g [2-(4-Chlorphenyl)-äthyl]-piperazin umgesetzt, wobei man das 1-{4-[N-(2-Isopropyloxyäthyl)-N-chloracetyl-amino]-benzoyl}-4-[2-(4-chlorphenyl)-äthyl]-piperazin vom Smp. 82-83⁰ erhält.

### Beispiel 59b:

Analog Beispiel 55 werden 1.5 g 4-[N-(2-Isopropyloxyäthyl)-N-chloracetyl-amino]-benzoylchlorid mit 0.76 g 2-Methylbenzyl-piperazin umgesetzt, wobei man das 1-{4-[N-(2-Isopropyloxyäthyl)-N-chloracetyl-amino]-benzoyl}-4-(2-methylbenzyl)-piperazin. Das Hydrochlorid davon schmilzt bei 142-143⁰.

### Beispiel 60:

Analog Beispiel 55 werden 1.5 g 4-[N-(2-Isopropyloxyäthyl)-N-chloracetyl-amino]-benzoylchlorid mit 0.81 g Cinnamyl-piperazin (Beispiel 27) umgesetzt, wobei man das 1-{4-[N-(2-Isopropyloxyäthyl)-N-chloracetyl-amino]-benzoyl}-4-[3-phenyl-prop-2-enyl]-piperazin erhält. Das Hydrochlorid davon schmilzt bei 188-190⁰.

### Beispiel 61:

Analog wie im Beispiel 22 beschrieben werden 1-{4-[N-(2-Isopropyloxyäthyl)-amino]-benzoyl}-4-[2-(4-chlorphenyl)-äthyl]-piperazin (Beispiel 1) und N,N-Dimethylglycinchlorid umgesetzt, wobei man das 1-{4-[N-(2-Isopropyloxyäthyl)-N,N-dimethylaminoacetyl-amino]-benzoyl}-4-[2-(4-chlorphenyl)-äthyl]-piperazin erhält. Das Difumarat davon schmilzt bei 187-188°.

### Beispiel 62:

Analog zu Beispiel 51 1-{4-[N-(2-Isopropyloxyäthyl)-N-chloracetyl-amino]-benzoyl}-4-[2-(4-chlorphenyl)-äthyl]-piperazin (Beispiel 3) und Piperidin umgesetzt, wobei man das 1-{4-[N-(2-Isopropyloxyäthyl)-N-piperidinoacetyl-amino]-benzoyl}-4-[2-(4-chlorphenyl)-äthyl]-piperazin erhält. Das Difumarat davon schmilzt bei 179-180°.

### Beispiel 63:

1.02 g 1-(2-Chlor-4-amino-benzoyl)-4-[2-(4-chlorphenyl)-äthyl]-piperazin (s. Europäische Patentanmeldung Nr. 489 690, Beispiel 7) in 50 ml abs. Tetrahydrofuran werden mit 0.33 g Chloracetylchlorid analog Beispiel 22 acyliert, wobei man das 1-[(2-Chlor-4-chloracetyl-amino)-benzoyl]-4-[2-(4-chlorphenyl)-äthyl]-piperazin-hydrochlorid vom Smp. 290-293⁰ erhält.

### Beispiel 64:

Analog zu Beispiel 63 wird 1-(4-Fluor-3-amino-benzoyl)-4-[2-(4-chlorphenyl)-äthyl]-piperazin mit Chloracetylchlorid umgesetzt, wobei man das 1-[(4-Fluor-3-chloracetyl-amino)-benzoyl]-4-[2-(4-chlorphenyl)-äthyl]-piperazin erhält.

### Beispiel 65:

Analog zu Beispiel 63 wird 1 -(2-Chlor-3-amino-benzoyl)-4-[2-(4-chlorphenyl)-äthyl]-piperazin mit Chloracetylchlorid umgesetzt, wobei man das 1-[(2-Chlor-3-chloracetyl-amino)-benzoyl]-4-[2-(4-chlorphenyl)-äthyl]-piperazin vom Smp. 156-157⁰ erhält.

### Beispiel 66:

3 g 4-Chlor-3-chloracetylamino-benzoesäurechlorid (hergestellt aus der entsprechenden Säure mit Thionylchlorid, Smp. 139-140⁰) in 12 ml abs. Tetrahydrofuran werden mit 1.75 g Hünig-Base und 2.59 g 1-[2-(4-Chlorphenyl)-äthyl]-piperazin in 40 ml Tetrahydrofuran bei 5⁰ tropfenweise versetzt. Nach 30 Min. Rühren bei RT wird das Gemisch eingedampft. Den Rückstand in Äther aufnehmen, mit Wasser waschen, einengen und den Niederschlag abnutschen. Man erhält so das 1-[(4-Chlor-3-chloracetylamino)-benzoyl]-4-[2-(4-chlorphenyl)-äthyl]-piperazin vom Smp. 64-65⁰.

### Beispiel 67:

Analog zu Beispiel 66 wird 3-Methoxy-4-chloracetylamino-benzoesäurechlorid mit 1-[2-(4-Chlorphenyl)-äthyl]-piperazin umgesetzt, wobei man das 1-[(3-Methoxy-4-chloracetyl-amino)-benzoyl]-4-[2-(4-chlorphenyl)-äthyl]-piperazin erhält.

### Beispiel 68:

Analog zu Beispiel 66 wird 3-Methyl-4-chloracetylamino-benzoesäurechlorid (Smp. 105-106°) mit 1-[2-(4-Chlorphenyl)-äthyl]-piperazin umgesetzt, wobei man das 1-[(3-Methyl-4-chloracetyl-amino)-benzoyl]-4-[2-(4-chlorphenyl)-äthyl]-piperazin vom Smp. 143-145⁰ erhält.

### Beispiel 69:

Analog zu Beispiel 29 werden aus den entsprechend substituierten Isatosäureanhydriden die folgenden Verbindungen synthetisiert:
(a) 1-[(3-Chlor-2-chloracetyl-amino)-benzoyl]-4-[2-(4-chlorphenyl)-äthyl]-piperazin
(b) 1-[(4-Chlor-2-chloracetyl-amino)-benzoyl]-4-[2-(4-chlorphenyl)-äthyl]-piperazin
(c) 1-[(5-Chlor-2-chloracetyl-amino)-benzoyl]-4-[2-(4-chlorphenyl)-äthyl]-piperazin, Smp. 140-141°.
(d) 1-[(3,5-Dichlor-2-chloracetyl-amino)-benzoyl]-4-[2-(4-chlorphenyl)-äthyl]-piperazin.

### Beispiel 70:

Analog zu Beispiel 2 und 3 werden die folgenden Verbindungen synthetisiert:
(a) 1-{4-[N-(2-Methoxyäthyl)-N-chloracetyl-amino]-benzoyl}-4-[2-(4-chlorphenyl)-äthyl] -piperazin
(b) 1-{4-[N-(2-(2-Methoxyäthoxy)-äthyl)-N-chloracetyl-amino]-benzoyl}-4-[2-(4-chlorphenyl)-äthyl]-piperazin
(c) 1-{4-[N-(2-n-Butyloxyäthyl)-N-chloracetyl-amino]-benzoyl}-4-[2-(4-chlorphenyl)-äthyl]-piperazin
(d) 1-{4-[N-(2-Aethoxyäthyl)-N-chloracetyl-amino]-benzoyl}-4-[2-(4-chlorphenyl)-äthyl]-piperazin
(e) 1-{4-[N-(2-Phenoxyäthyl)-N-chloracetyl-amino]-benzoyl}-4-[2-(4-chlorphenyl)-äthyl]-piperazin
(f) 1-{4-[N-(3-Methoxypropyl)-N-chloracetyl-amino]-benzoyl}-4-[2-(4-chlorphenyl)-äthyl]-piperazin.

### Beispiel 71:

Analog zu Beispiel 7 werden 1-[2-(4-Chlorphenyl)-äthyl]-piperazin und 4-Chloracetylamino-zimtsäurechlorid zum 1-{3-[4-(N-Chloracetyl-amino)-phenyl]-prop-2-en-1-on-yl}-4-[2-(4-chlorphenyl)-äthyl]-piperazin umgesetzt.

### Beispiel 72:

Tabletten, enthaltend je 50 mg 1-{4-[N-(2-Isopropyloxyäthyl)-N-chloracetylamino]-benzoyl}-4-[2-(4-chlorphenyl)-äthyl]-piperazin oder ein Salz, z.B. das Hydrochlorid, werden z.B. wie folgt hergestellt:

### Zusammensetzung (10000 Tabletten)

- Wirkstoff: 500,0 g
- Lactose: 500,0 g
- Kartoffelstärke: 352,0 g
- Gelatine: 8,0 g
- Talk: 60,0 g
- Magnesiumstearat: 10,0 g
- Siliciumdioxid (hochdispers): 20,0 g
- Äthanol: q.s.

Der Wirkstoff wird mit der Lactose und 292 g kartoffelstärke vermischt, die Mischung mit einer äthanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

### Beispiel 73:

Gelatinesteckkapseln, enthaltend 100 mg Wirkstoff, z.B. 1-{4-[N-(2-Isopropyloxyäthyl)-N-chloracetyl-amino]-benzoyl}-4-[2-(4-chlorphenyl)-äthyl]-piperazin oder ein Salz davon, z.B. das Hydrochlorid, werden z.B. wie folgt hergestellt:

### Zusammensetzung (für 1000 Kapseln)

- Wirkstoff: 100,0 g
- Lactose: 250,0 g
- mikrokristalline Zellulose: 30,0 g
- Natriumlaurylsulfat: 2,0 g
- Magnesiumstearat: 8,0 g.

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteckkapseln der Grösse 0 abgefüllt.

### Beispiel 74:

Lacktabletten, enthaltend je 100 mg 1-{4-[N-(2-Isopropyloxyäthyl)-N-chloracetyl-amino]-benzoyl}-4-[2-(4-chlorphenyl)-äthyl]-piperazin oder ein Salz, z.B. das Hydrochlorid, davon werden z.B. wie folgt hergestellt:

### Zusammensetzung (für 1000 Lacktabletten)

- Wirkstoff: 100,0 g
- Lactose: 100,0 g
- Maisstärke: 70,0 g
- Talk: 8,5 g
- Calciumstearat: 1,5 g
- Hydroxypropylmethylcellulose: 2,36 g
- Schellack: 0,64 g
- Wasser: q.s.
- Methylenchlorid: q.s. .

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

### Beispiel 75:

Eine 0,2%-ige Injektions- oder Infusionslösung von 1-{4-[N-(2-Isopropyloxyäthyl)-N-chloracetyl-amino]-benzoyl}-4-[2-(4-chlorphenyl)-äthyl]-piperazin oder eines Salzes, z.B. des Hydrochlorides, davon werden z.B. wie folgt hergestellt:

### Zusammensetzung (für 1000 Ampullen)

- Wirkstoff: 5,0 g
- Natriumchlorid: 22,5 g
- Phosphatpuffer pH= 7.4: 300,0 g
- entmineralisiertes Wasser: ad 2500,0 ml

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

### Beispiel 76:

In analoger Weise wie in Beispiel 72 bis 75 beschrieben, können auch pharmazeutische Präparate, die jeweils eine andere der in den Beispielen 1 bis 71 genannten Verbindungen enthalten, hergestellt werden.

## Patentansprüche

1. Verbindungen der Formel I, 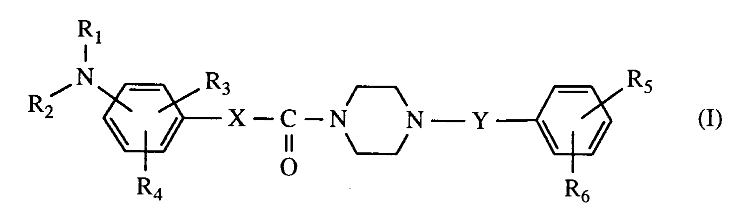 worin R₁ für Wasserstoff, Niederalkyl, Niederalkoxy-niederalkyl, Niederalkoxy-niederalkoxy-niederalkyl, Aryloxy-niederalkyl, Arylniederalkoxy-niederalkyl, N-Niederalkylamino-niederalkyl oder N,N-Diniederalkylamino-niederalkyl steht, R₂ Niederalkenoyl, (Carboxy oder Niederalkoxycarbonyl)-niederalkenoyl, elektronegativ substituiertes Niederalkanoyl, Carbamoyl, N-Niederalkylcarbamoyl, N,N-Diniederalkylcarbamoyl, Carboxy-carbonyl, Niederalkoxycarbonyl-carbonyl, (Carbamoyl, N-Niederalkylcarbamoyl oder N,N-Diniederalkylcarbamoyl)-carbonyl bedeutet oder R₂, sofern R₁ für Niederalkoxy-niederalkyl, Niederalkoxy-niederalkoxy-niederalkyl, Aryloxy-niederalkyl, Arylniederalkoxy-niederalkyl, N-Niederalkylamino-niederalkyl oder N,N-Diniederalkylamino-niederalkyl steht, auch Wasserstoff, Niederalkanoyl, Carboxy, Niederalkoxycarbonyl oder Arylniederalkoxycarbonyl bedeuten kann, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, und X und Y unabhängig voneinander eine direkte Bindung, Niederalkylen oder Niederalkenylen bedeuten, und Salze davon.

2. Verbindungen der Formel I gemäss Anspruch 1, worin R₁ für Wasserstoff, Niederalkyl, Niederalkoxy-niederalkyl, Niederalkoxy-niederalkoxy-niederalkyl; Phenyloxy-niederalkyl oder Phenylniederalkoxy-niederalkyl, wobei in den beiden letztgenannten Resten die Phenylgruppe jeweils unsubstituiert oder durch Niederalkyl, Trifluormethyl, Niederalkoxy, Halogen und/oder Hydroxy substituiert ist; N-Niederalkylamino-niederalkyl oder N,N-Diniederalkylamino-niederalkyl steht, R₂ Niederalkenoyl, (Carboxy oder Niederalkoxycarbonyl)-niederalkenoyl, Halogen-niederalkanoyl, Amino-niederalkanoyl, N-Niederalkylamino-niederalkanoyl, N-[(Carboxy oder Niederalkoxycarbonyl)-niederalkylamino]-niederalkanoyl, N,N-Diniederalkylamino-niederalkanoyl, C₄-C₆-Niederalkylenamino-niederalkanoyl, Morpholino-niederalkanoyl, Thiomorpholino-niederalkanoyl; Piperazino-niederalkanoyl, wobei der Piperazinorest unsubstituiert oder in 4-Stellung durch Niederalkyl oder Niederalkanoyl substituiert ist; Hydroxy-niederalkanoyl, Niederalkoxy-niederalkanoyl, Niederalkanoyloxy-niederalkanoyl, Niederalkylthio-niederalkanoyl, Niederalkylsulfinyl-niederalkanoyl, Niederalkylsulfonyl-niederalkanoyl; Phenylthio-niederalkanoyl, Phenylsulfinyl-niederalkanoyl oder Phenylsulfonyl-niederalkanoyl, wobei in den drei letztgenannten Resten die Phenylgruppe jeweils unsubstituiert oder durch Niederalkyl, Niederalkoxy, Halogen und/oder Hydroxy substituiert ist; Carboxy-niederalkanoyl, Niederalkoxycarbonyl-niederalkanoyl, Carbamoyl-niederalkanoyl, N-Niederalkylcarbamoyl-niederalkanoyl, N,N-Diniederalkylcarbamoyl-niederalkanoyl, Cyano-niederalkanoyl, Carbamoyl, N-Niederalkylcarbamoyl, N,N-Diniederalkylcarbamoyl, Carboxy-carbonyl, Niederalkoxycarbonyl-carbonyl, (Carbamoyl, N-Niederalkylcarbamoyl oder N,N-Diniederalkylcarbamoyl)-carbonyl bedeutet oder R₂, sofern R₁ für Niederalkoxy-niederalkyl, Niederalkoxy-niederalkoxy-niederalkyl, unsubstituiertes oder wie oben definiert substituiertes Phenyloxy-niederalkyl oder Phenylniederalkoxy-niederalkyl, N-Niederalkylamino-niederalkyl oder N,N-Diniederalkylamino-niederalkyl steht, auch Wasserstoff, Niederalkanoyl, Carboxy, Niederalkoxycarbonyl oder Phenylniederalkoxycarbonyl, worin die Phenylgruppe unsubstituiert oder durch Niederalkyl, Niederalkoxy, Halogen und/oder Hydroxy substituiert ist, bedeuten kann, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, und X und Y unabhängig voneinander eine direkte Bindung, Niederalkylen oder Niederalkenylen bedeuten, und Salze davon.

3. Verbindungen der Formel I gemäss Anspruch 1, worin R₁ für Wasserstoff, Niederalkyl, Niederalkoxy-niederalkyl, Niederalkoxy-niederalkoxy-niederalkyl, Phenyloxy-niederalkyl, N-Niederalkylamino-niederalkyl oder N,N-Diniederalkylamino-niederalkyl steht, R₂ Niederalkenoyl, (Carboxy oder Niederalkoxycarbonyl)-niederalkenoyl, Halogen-niederalkanoyl, N-Niederalkylamino-niederalkanoyl, N-[(Carboxy oder Niederalkoxycarbonyl)-niederalkylamino]-niederalkanoyl, N,N-Diniederalkylamino-niederalkanoyl, Piperidino-niederalkanoyl, Hydroxy-niederalkanoyl, Niederalkoxy-niederalkanoyl, Niederalkanoyloxy-niederalkanoyl, Niederalkylthio-niederalkanoyl, Niederalkylsulfinyl-niederalkanoyl, Niederalkylsulfonyl-niederalkanoyl, Phenylthio-niederalkanoyl, Phenylsulfinyl-niederalkanoyl, Phenylsulfonyl-niederalkanoyl, Carboxy-niederalkanoyl, Niederalkoxycarbonyl-niederalkanoyl, N,N-Diniederalkylcarbamoyl oder Niederalkoxycarbonyl-carbonyl bedeutet oder R₂, sofern R₁ für Niederalkoxy-niederalkyl oder N,N-Diniederalkylamino-niederalkyl steht, auch Wasserstoff, Niederalkanoyl, Niederalkoxycarbonyl oder Phenylniederalkoxycarbonyl bedeuten kann, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Trifluormethyl, Niederalkoxy, Niederalkylthio, Halogen oder Diniederalkylamino stehen, und X und Y unabhängig voneinander eine direkte Bindung, Niederalkylen oder Niederalkenylen bedeuten, und Salze davon.

4. Verbindungen der Formel I gemäss Anspruch 1, worin R₁ für Wasserstoff, Niederalkyl oder Niederalkoxy-niederalkyl steht, R₂ Niederalkenoyl oder Halogen-niederalkanoyl bedeutet oder R₂, sofern R₁ für Niederalkoxy-niederalkyl steht, auch Wasserstoff bedeuten kann, R₃ und R₄ Wasserstoff bedeuten, R₅ für Chlor steht, R₆ Wasserstoff bedeutet, X für eine direkte Bindung steht und Y 1,2-Aethylen bedeutet, und pharmazeutisch verwendbare Salze davon.

5. Verbindungen gemäss Anspruch 1 von der Formel Ia, 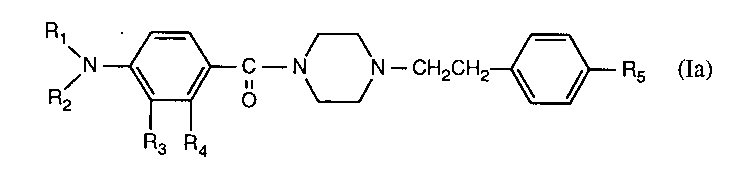 worin R₁ für Wasserstoff, Niederalkyl oder Niederalkoxyniederalkyl steht, R₂ Niederalkenoyl oder durch Halogen, Diniederalkylamino, 4- bis 6-gliedriges Niederalkylenamino oder Niederalkylthio substituiertes Niederalkanoyl bedeutet oder R₂, sofern R₁ Niederalkoxyniederalkyl darstellt, Wasserstoff bedeuten kann, R₃ und R₄ unabhängig voneinander Wasserstoff, Niederalkyl, Chlor, Fluor oder Brom bedeuten und R₅ Niederalkylthio, Chlor, Fluor oder Brom darstellt, und Salze davon.

6. Verbindungen der Formel Ia gemäss Anspruch 5, worin R₁ Wasserstoff, Niederalkyl oder Niederalkoxyniederalkyl bedeutet, R₂ für durch Halogen, Niederalkylthio, Niederalkylsulfinyl oder Niederalkylsulfonyl substituiertes Niederalkanoyl oder, sofern R₁ Niederalkoxyniederalkyl bedeutet, für Wasserstoff stehen kann, R₃ und R₄ unabhängig voneinander Wasserstoff, Niederalkyl, Chlor, Fluor oder Brom bedeuten und R₅ Niederalkylthio, Chlor, Fluor oder Brom darstellt, und ihre Salze.

7. Verbindungen der Formel Ia gemäss Anspruch 5, worin R₁ Wasserstoff, C₁-C₄-Alkyl oder C₁-C₄-Alkoxy-C₂-C₄-alkyl bedeutet, R₂ C₃-C₇-Alkenoyl, Chlor-C₂-C₄-alkanoyl, C₁-C₄-Alkylthio-, C₁-C₄-Alkylsulfinyl- oder C₁-C₄-Alkylsulfonyl-C₂-C₄-alkanoyl oder, sofern R₁ C₁-C₄-Alkoxy-C₂-C₄-alkyl bedeutet, Wasserstoff darstellen kann, R₃ und R₄ unabhängig voneinander Wasserstoff, C₁-C₄-Alkyl, Fluor oder Chlor bedeuten und R₅ für Chlor steht, und pharmazeutisch verwendbare Salze davon.

8. Verbindungen der Formel I gemäss Anspruch 1, worin R₁ Wasserstoff, Methyl, Äthyl oder 2-Isopropyloxyäthyl bedeutet, R₂ Prop-2-enoyl, Chloracetyl, 3-Chlorpropionyl, Methylthioacetyl oder Äthylthioacetyl darstellt, R₃ und R₄ Wasserstoff, Methyl, Fluor oder Chlor bedeuten und R₅ für Chlor steht, und pharmazeutisch verwendbare Salze davon.

9. 1-{4-[N-(2-Isopropyloxyäthyl)-amino]-benzoyl}-4-[2-(4-chlorphenyl)-äthyl]-piperazin gemäss Anspruch 1, oder ein pharmazeutisch anwendbares Salz davon.

10. 1-{4-[N-(2-Isopropyloxyäthyl)-N-chloracetyl-amino]-benzoyl}-4-[2-(4-chlorphenyl)-äthyl]-piperazin gemäss Anspruch 1, oder ein pharmazeutisch anwendbares Salz davon.

11. 1-[4-(Acryloylamino)-benzoyl]-4-[2-(4-chlorphenyl)-äthyl]-piperazin gemäss Anspruch 1, oder ein pharmazeutisch anwendbares Salz davon.

12. Pharmazeutische Präparate enthaltend eine Verbindung gemäss einem der Ansprüche 1 bis 11 und mindestens ein pharmazeutisch verwendbares Trägermaterial.

13. Eine Verbindung gemäss einem der Ansprüche 1 bis 11 zur Anwendung in einem Verfahren zur therapeutischen Behandlung des tierischen oder menschlichen Körpers.

14. Eine Verbindung gemäss einem der Ansprüche 1 bis 11 zur Verwendung bei der Behandlung von Krankheiten, die auf eine Hemmung von Interleukin-1 ansprechen.

15. Verwendung einer Verbindung gemäss einem der Ansprüche 1 bis 11 zur Herstellung pharmazeutischer Präparate.

16. Verwendung einer Verbindung gemäss einem der Ansprüche 1 bis 11 zur Herstellung pharmazeutischer Präparate für die Behandlung von Krankheiten, die auf eine Hemmung von Interleukin-1 ansprechen.

17. Verfahren zur Herstellung einer Verbindung der Formel I gemäss Anspruch 1, dadurch gekennzeichnet, dass man
a) eine Verbindung der Formel II, 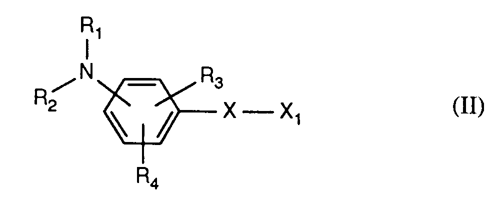 worin X₁ Carboxy oder reaktionsfähiges funktionell abgewandeltes Carboxy bedeutet, oder ein Salz davon, mit einer Verbindung der Formel III, 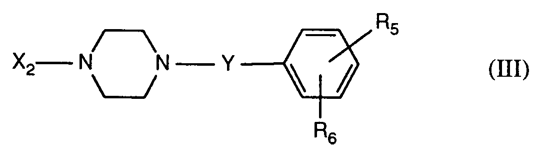 worin X₂ Wasserstoff oder eine Aminoschutzgruppe bedeutet, umsetzt, oder
b) eine Verbindung der Formel IV, 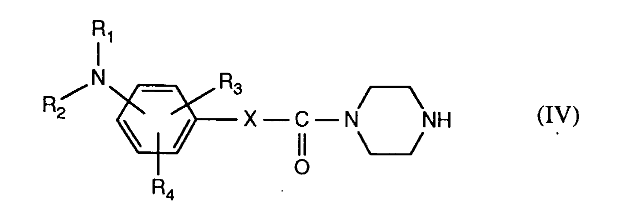 oder ein Salz davon, mit einer Verbindung der Formel V, 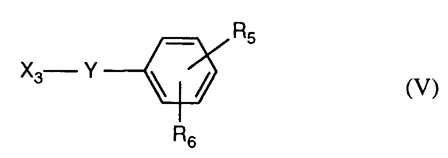 worin X₃ Hydroxy oder reaktionsfähiges verestertes Hydroxy bedeutet, umsetzt, oder
c) eine Verbindung der Formel VI, 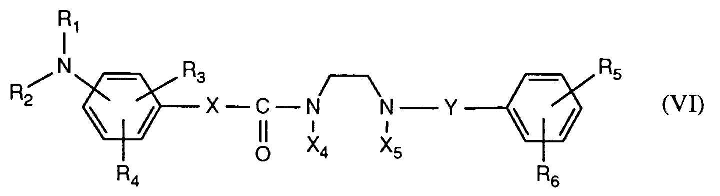 worin einer der Reste X₄ und X₅ Wasserstoff und der andere eine Gruppe der Formel -CH₂-CH₂-X₃ (VIa) bedeutet und X₃ für Hydroxy oder reaktionsfähiges verestertes Hydroxy steht, cyclisiert, oder
d) eine Verbindung der Formel VII, 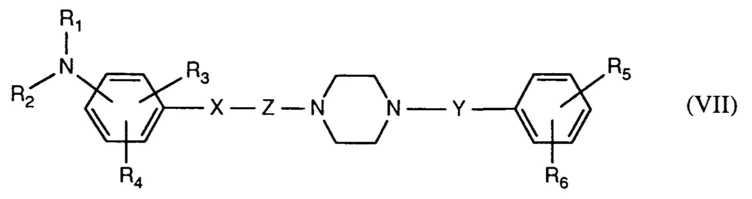 worin Z eine zu Carbonyl oxidierbare Gruppe bedeutet, oxidiert, oder
e) in eine Verbindung der Formel VIII, 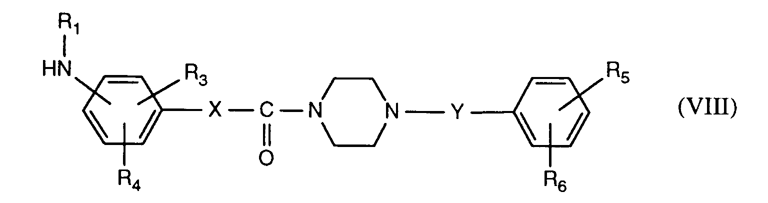 den Rest R₂ einführt (R₂ ≠ H), oder
f) zur Herstellung von Verbindungen, worin R₁ Niederalkoxyniederalkyl bedeutet, in eine Verbindung der Formel IX 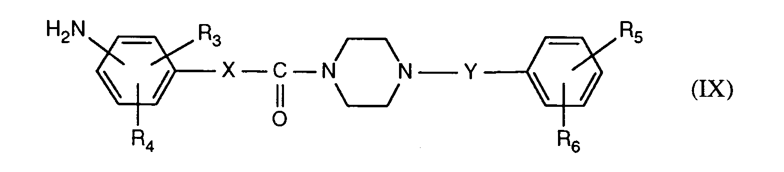
den Rest R₁ einführt; und jeweils gewünschtenfalls eine verfahrensgemäss oder auf andere Weise erhältliche Verbindung in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensgemäss erhältliches Salz in die freie Verbindung der Formel I oder in ein anderes Salz überführt.
